# EUROPEAN PATENT SPECIFICATION

(11) **EP 0 905 519 B1**
(45) Date of publication and mention of the grant of the patent: **25.05.2005**
(21) Application number: 97306936.2
(22) Date of filing: 08.09.1997
(51) Int. Cl.: G01N 33/68, C07K 1/12

(54) **A method for amino acid sequencing of protein or peptide from carboxy terminus thereof**
Verfahren zur Bestimmung der Sequenz von Aminosäuren von Proteinen oder Peptiden, beginnend mit dem Carboxylende
Procédé pour la détermination de la séquence des acides aminés de protéines ou de peptides, commençant par la terminaison carboxyle

(43) Date of publication of application: 31.03.1999
(73) Proprietor: SEIKO INSTRUMENTS INC., Chiba-shi, Chiba 261 (JP)
(72) Inventor: Tsugita, Akira, Kashiwa-shi, Chiba (JP); Takamoto, Keiji, Nagareyama-shi, Chiba (JP); Ataka, Tatsuaki, Mihama-ku, Chiba-shi, Chiba (JP); Sakuhara, Toshihiko, Mihama-ku, Chiba-shi, Chiba (JP); Uchida, Toyoaki, Mihama-ku, Chiba-shi, Chiba (JP)
(74) Representative: Sturt, Clifford Mark

(56) References cited:
- WO-A-95/03066
- JP-A- 6 027 113
- US-A- 5 180 807
- US-A- 5 468 843
- BAILEY, JEROME M. ET AL: "Automated C-terminal sequencing of peptides" TECH. PROTEIN CHEM. 3, [PAP. ANNU. SYMP. PROTEIN SOC.], 5TH (1992), MEETING DATE 1991, 11-21. EDITOR(S): ANGELETTI, RUTH HOGUE. PUBLISHER: ACADEMIC, SAN DIEGO, CALIF. CODEN: 57VOAN, XP002056916
- BASU, GAUTAM ET AL: "Conformational preferences of oligopeptides rich in.alpha.-aminoisobutyric acid. I. Observation of a 310/.alpha.-helical transition upon sequence permutation" BIOPOLYMERS (1991), 31(14), 1763-74 CODEN: BIPMAA;ISSN: 0006-3525, XP002056915

## Description

The present invention relates to a method for analysing the primary structure of a protein or a peptide, and particularly relates to a method for amino acid sequencing of a protein or a peptide from a carboxy terminus thereof.

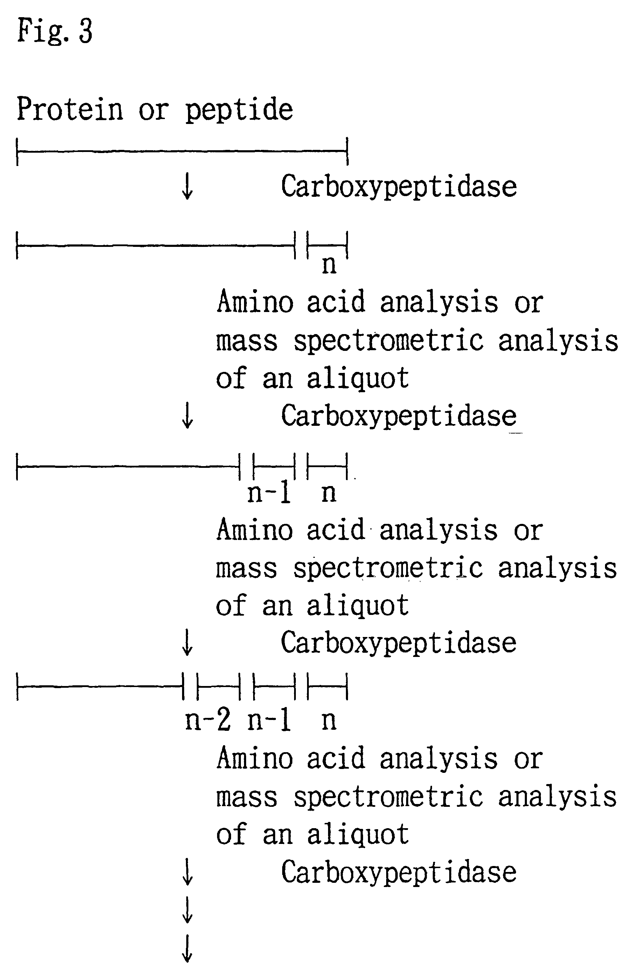

In order to determine an amino acid sequence of a protein or a peptide from a carboxy terminus thereof (hereinafter sometimes referred to as a C-terminal), a method, as shown in Fig. 3, comprising of allowing a protein or a peptide to react with carboxypeptidase, collecting portions of the reaction mixture with the passage time, analysing the reaction mixture by an amino acid analyser, and analysing the liberated amino acid has been used (Seikagakujikkenkouza Vol.1, Tanpakushitunokagaku II, edited b; Nihonseikagakukai, p203-211, 1976).

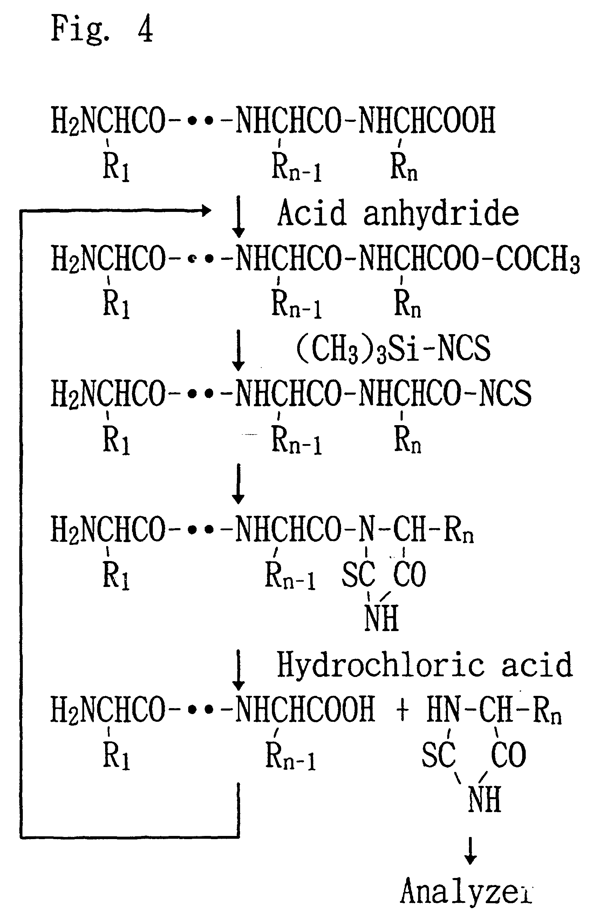

A method for analysing the mass of a C-terminal truncated protein or a C-terminal truncated peptide by analysing the reaction mixture using a mass spectrograph is reported (A. Tsugita, R. van den Broek, M. Pyzybylski, FEBS. Lett. 137, 19(1982)). Also, as an example of the chemical methods, a method for analysing a sequence comprising repeating a series of operations consisting of activating the C-terminal with acetic anhydride, binding trimethylsilylisothiocyanate, and cleaving with hydrochloric acid is reported as shown in Fig. 4 (D. H. Hawke, H-. W. Lahm, J. F. Shively, C. W. Todd, Anal. Biochem. 166, 298(1987)).

The conventional method using carboxypeptidase has difficulty in accurate determination by cleavage at undesirable peptide bonds derived from the diversity of substrate specificity and activity of the enzyme depending upon the C-terminal amino acid or an adjacent amino acid thereof, and from the contamination of the other protease. Also this method is not suitable for microanalysis, since the contamination by the mixture therein of amino acid and peptide occurs because of the reasons that the enzyme has an autolytic property and is unsatisfactorily purified sometimes.

Further, the other chemical method is not sufficiently made to a practical use.

It is an object of the present invention to provide a chemical method for amino acid sequencing of a protein or a peptide from a C-terminus thereof without using enzymes.

It is another object of the present invention to provide a chemical method being suitable for microanalysis of amino acid sequencing of a protein or peptide.

According to a first aspect of the present invention, there is provided a method for amino acid sequencing of a protein or a peptide from a carboxy terminus thereof comprising repeating a combined procedure including a process of liberating a carboxy-terminal amino acid of a protein or a peptide and an operation of isolating and identifying the amino acid or amino acid derivative obtained, wherein said combined procedure comprises a first process of allowing the protein or the peptide to react with an acid anhydride and modifying the carboxy terminus to generate oxazolone, including protecting the amino terminal in the operation of said first process; and a second process of allowing the protein or the peptide generated, of which the carboxy terminus is oxazolone, to react with a) acid and alcohol, b) a solution containing acid and alcohol, or c) an ester, to liberate the carboxy-terminal amino acid and an operation of isolating and identifying the amino acid obtained in the second process.

According to a second aspect of the present invention, there is provided a method for amino acid sequencing of a protein or a peptide from a carboxy terminus thereof, characterised by repeatedly carrying out a combined procedure of a series of operations comprising a first process of allowing a protein or a peptide to react with an acid anhydride, protecting an amino group thereof, and modifying the carboxy terminus to generate oxazolone when the carboxy-terminal amino acid is not aspartic acid or to give an intramolecular acid anhydride when it is aspartic acid, a second process of reacting with an alcohol in the presence of acid to liberate the carboxy-terminal amino acid when the carboxy terminal is oxazolone or to ring-open the intramolecular acid anhydride by alcoholysis when it is an intramolecular acid anhydride, a third process of reacting with an acid anhydride to form oxazolone at the carboxy terminal, a fourth process of reacting with an alcohol to liberate the carboxy-terminal amino acid as an ester of the amino acid, and a fifth process of reacting with an amine to hydrolyze the ester and an operation of isolating and identifying a) the amino acid obtained in the second process, and/or the fifth process, or b) the amino acid derivative obtained in the third, fourth or fifth process.

The invention will now be described by way of example with reference to the accompanying drawings, in which:-
Fig. 1 shows a first flow chart representing a method for analysing amino acid sequence of the present invention;
Fig. 2 shows a reaction scheme illustrating an experimental procedure of the present invention;
Fig. 3 shows a flow chart representing a conventional determination method using carboxypeptidase;
Fig. 4 shows a flow chart representing a conventional determination method using trimethylsilylisothiocyanate;
Fig. 5 shows a drawing showing a result of HPLC analysis to confirm the retention time of alanyltryptophan;
Fig. 6 shows a drawing showing a result of HPLC analysis to confirm the retention time of N-acetylalanyltryptophan;
Fig. 7 shows a drawing showing a result of HPLC analysis to confirm the retention time of N-acetylalanyltryptophanylmethionylarginylphenylalanine;
Fig. 8 shows a drawing showing a result of HPLC analysis to confirm the retention time of N-acetylalanyltryptophanylmethionylarginylphenylalanylalanine;
Fig. 9 shows a drawing showing a result of HPLC analysis to confirm generation of oxazolone;
Fig. 10 shows a drawing showing a result of HPLC analysis to confirm cleavage of the oxazolone ring;
Fig. 11 shows a second flow chart representing a method for analysing amino acid sequence of the present invention;
Fig. 12 shows a reaction scheme illustrating another experimental procedure of the present invention;
Fig. 13 shows a drawing showing a result of HPLC analysis to confirm the retention time of tryptophan;
Fig. 14 shows a drawing showing a result of HPLC analysis to confirm the retention time of alanyltryptophan;
Fig. 15 shows a drawing showing a result of HPLC analysis to confirm the retention time of N-acetylalanyltryptophan;
Fig. 16 shows a drawing showing a result of HPLC analysis to confirm the retention time of the ethyl ester of N-acetylalanyltryptophan;
Fig. 17 shows a drawing showing a result of HPLC analysis to confirm generation of oxazolone;
Fig. 18 shows a drawing showing a result of HPLC analysis to confirm cleavage of the oxazolone ring;
Fig. 19 shows a drawing showing a result of HPLC analysis to confirm hydrolysis;
Fig. 20 shows a drawing showing a result of mass spectrometric analysis of the sample obtained in Example 8;
Fig. 21 shows a drawing showing a result of mass spectrometric analysis of the sample obtained in Example 9;
Fig. 22 shows a drawing showing a result of mass spectrometric analysis of the sample obtained in Example 10;
Fig. 23 shows a drawing showing a result of mass spectrometric analysis of the sample obtained in Example 11;

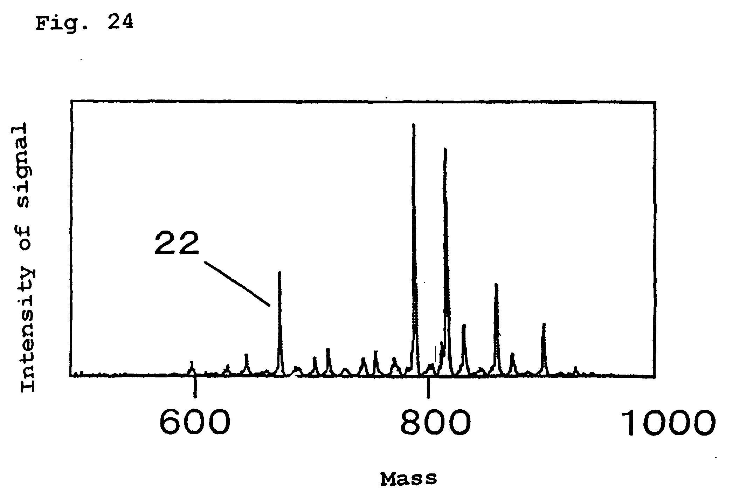

Fig. 24 shows a drawing showing a result of mass spectrometric analysis of the sample obtained in Example 12;
Fig. 25 shows a drawing showing a result of mass spectrometric analysis of the sample obtained in Example 13;
Fig. 26 shows a drawing showing a result of mass spectrometric analysis of the sample obtained in Example 14;
Fig. 27 shows a drawing showing a result of mass spectrometric analysis of the sample obtained in Example 15;
Fig. 28 shows a drawing showing a result of mass spectrometric analysis of the sample obtained in Example 16;
Fig. 29 shows a drawing showing a result of mass spectrometric analysis of the sample obtained in Example 17;
Fig. 30 shows a drawing showing a result of mass spectrometric analysis of the sample obtained in Example 18;
Fig. 31 shows a drawing showing a result of mass spectrometric analysis of the sample obtained in Example 19;
Fig. 32 shows a drawing showing a result of mass spectrometric analysis of the sample obtained in Example 20;
Fig. 33 shows a drawing showing a result of mass spectrometric analysis of the sample obtained in Example 21;
Fig. 34 shows a drawing showing a result of mass spectrometric analysis of the sample obtained in Example 22;
Fig. 35 shows a drawing showing a result of mass spectrometric analysis of the sample obtained in Example 23;
Fig. 36 shows a third flow chart representing a method for analysing amino acid sequence of the present invention;
Fig. 37 shows a reaction scheme illustrating another experimental procedure of the present invention;
Fig. 38 shows a drawing illustrating another experimental procedure of the present invention;
Fig. 39 shows a drawing showing a result of mass spectrometric analysis to confirm generation of oxazolone;
Fig. 40 shows a drawing showing a result of mass spectrometric analysis to confirm cleavage of the oxazolone ring;
Fig. 41 shows a drawing showing a result of HPLC analysis to confirm cleavage of the oxazolone ring;
Fig. 42 shows a drawing showing a result of mass spectrometric analysis to confirm cleavage of the oxazolone ring of the product separated by HPLC;
Fig. 43 shows a drawing showing a result of mass spectrometric analysis to confirm cleavage of the oxazolone ring of the product separated by HPLC;
Fig. 44 shows a drawing showing a result of mass spectrometric analysis to confirm cleavage of the oxazolone ring of the product separated by HPLC; and
Fig. 45 shows a drawing showing a result of mass spectrometric analysis to confirm hydrolysis.

As a procedure for carrying out the present invention, a combination is repeated which comprises an operation consisting of a first and a second processes described below and an operation of isolating and identifying amino acid prepared.

### First Process

A protein or a peptide is allowed to react with an acid anhydride, an amino terminal of the protein or the peptide is protected, and an amino acid residue at a C-terminal is modified to generate oxazolone.

### Second Process

A protein or a peptide generated, whose carboxy terminal is oxazolone, is allowed to react with an acid and alcohol to liberate the carboxy-terminal amino acid.

As another example of the procedure, in order to overcome the problems described above and sequentially determine an amino acid from a C-terminus thereof, a combination is repeated which comprises a series of operations consisting of the respective processes of the first and second processes described above wherein acetic acid is added in the first process and an operation of isolating and identifying the amino acid obtained.

As another procedure for carrying out the present invention, a combination is repeated which comprises a series of the operations consisting of the respective processes of a first process to a third process described below and an operation of isolating and identifying an amino acid generated.

### First Process

A protein or a peptide is allowed to react with an acid anhydride, an amino group of the protein or the peptide is protected, and an amino acid residue at a C-terminal thereof is modified to generate oxazolone.

### Second Process

The reaction product in the first process is allowed to react with an alcohol in the presence of acid, the oxazolone contained therein is subjected to alcoholysis (esterification), and the carboxy-terminal amino acid is liberated.

### Third Process

The reaction product in the second process is allowed to react with an amine, the ester contained therein is hydrolyzed.

As still anotherexample of the procedure, a combination is repeated which comprises a series of the operations consisting of the respective processes of the first process to the third process described above wherein acetic acid is added in the first process and an operation of isolating and identifying the amino acid generated.

As yet another procedure for carrying out the present invention, a combination is repeated which comprises an operation consisting of a first process to a fifth process described below and an operation of isolating and identifying the amino acid or the amino acid derivative generated.

### First Process

A protein or a peptide is allowed to react with an acid anhydride, an amino terminal of the protein or the peptide is protected, and an amino acid residue at a C-terminal thereof is modified to generate oxazolone or an intramolecular acid anhydride (when the C-terminal amino acid is aspartic acid).

### Second Process

The reaction product in the first process is allowed to react with an alcohol in the presence of acid, the oxazolone contained therein is subjected to alcoholysis (esterification), and the carboxy-terminal amino acid is liberated or the intramolecular acid anhydride is ring-opened by alcoholysis.

### Third Process

The reaction product in the second process is allowed to react with an acid anhydride, the amino acid residue at the C-terminus of the protein or the peptide, whose C-terminal is not esterified, contained therein is modified to generate oxazolone.

### Fourth Process

The reaction product in the third process is allowed to react with an alcohol in the presence of acid, the oxazolone contained therein is subjected to alcoholysis (esterification), and the carboxy-terminal amino acid is liberated.

### Fifth Process

The reaction product in the fourth process is allowed to react with an amine and the ester contained therein is hydrolyzed.

As yet further another example of the procedure above, a combination is repeated which comprises a series of the operations consisting of the respective processes of the first process to the third process described above wherein the second and fourth processes to react with an alcohol are carried out in the presence of acid and an operation of isolating and identifying the amino acid or the amino acid derivative generated.

The present invention will be further described with reference to the following examples.

### Example 1

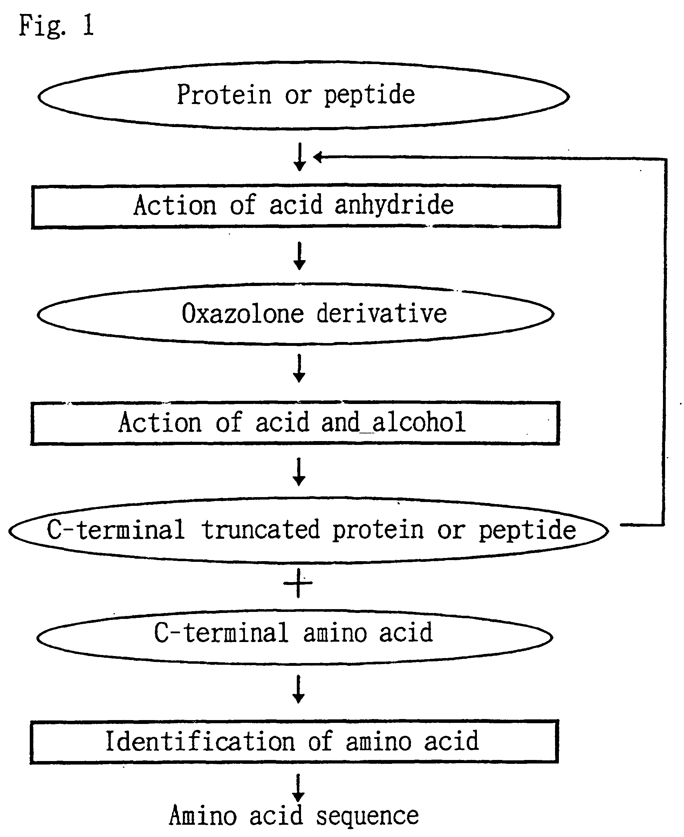

Fig. 1 shows a flow chart illustrating the present invention.

### First Process

A protein or a peptide is allowed to react with an anhydride of an organic acid represented by the general formula;

CH₃-(CH₂)m-CO-O-CO-(CH₂)n-CH₃

(m and n is an integer of 0 or above, respectively) to give a protein or a peptide whose amino group is modified and C-terminal amino acid converted into oxazolone.

### Second Process

Subsequently, the protein or the peptide, whose amino group is modified and C-terminal amino acid is converted into oxazolone, is allowed to react with an acid and alcohol to give a mixture of a protein or a peptide whose amino group is modified and one amino acid residue at the C-terminus is lacking and the original C-terminal amino acid.

The amino acid obtained thereby is isolated and identified to determine the C-terminal amino acid of the protein or the peptide.

Then the operations of the first and second processes using the protein or the peptide whose amino group is modified and one amino acid residue at the C-terminus is lacking, and the operation of isolating and identifying the amino acid obtained are repeatedly carried out to analyse the amino acid sequence of the protein or the peptide from the C-terminus.

### Example 2

Here, an example of the operation procedure of the present invention is described which analyses an amino acid sequence of a protein or a peptide from a C-terminus thereof by repeating operations of the first and second processes using a protein or a peptide and an operation of isolating and identifying the amino acid from the mixture obtained in the second process.

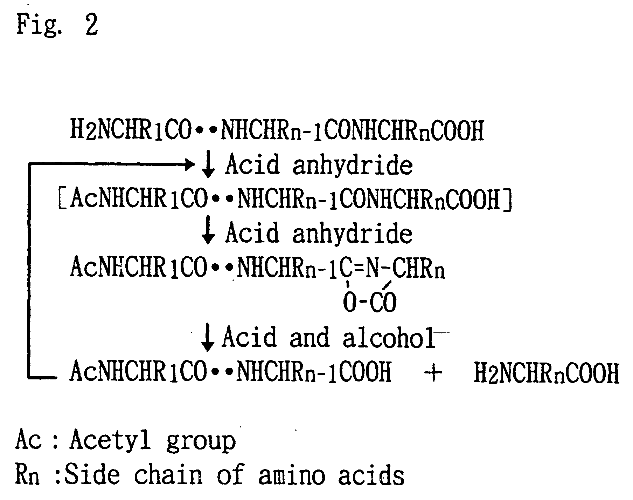

Fig. 2 shows a reaction scheme illustrating the experimental procedure of the present invention.

### First Process

A sample is allowed to react with acetic anhydride. The reaction conditions are as follows.

| Reaction conditions | |
|---|---|
| Concentration of acetic anhydride | 20% |
| (acetonitrile solution, acetic acid added to adjust to 1 % of the concentration) | |
| Reaction temperature | 60°C |
| Reaction period | 30 minutes |

After this reaction, the reaction mixture is evaporated under reduced pressure to dryness to remove the reagent and solvent.

### Second Process

The sample obtained is allowed to react with pentafluoropropionic acid (PFPA) and methanol. The reaction conditions are as follows.

| Reaction conditions | |
|---|---|
| Concentration of PFPA | 50% (methanol solution) |
| Reaction temperature | 60°C |
| Reaction period | 30 minutes |

Under these conditions, an ester, methylpentafluoropropionate is generated by the reaction of PFPA with methanol.

Then the reaction mixture is evaporated under reduced pressure to dryness to remove the reagent and solvent.

After the second process, the amino acid is isolated from the sample obtained and identified.

Then a combination of the procedures consisting of the first and second processes using the protein or the peptide whose amino group is modified and one amino acid residue at the C-terminus is lacking and the operation of isolating and identifying the amino acid obtained in the second process is repeatedly carried out to analyse the amino acid sequence of the protein or the peptide from the C-terminus.

### Example 3

Here, the conditions for analysis by high precision liquid chromatography (hereinafter referred to as HPLC) describing the present invention and the results of analysing standard compounds by HPLC are described.

The conditions for the HPLC analysis are as follows.

| Analysis conditions | |
|---|---|
| Column | Shiseido C18 MICROBORE |
| Elution | Gradient elution with the following Solution A and B |
| Solution A | 0.1%. TFA aqueous solution |
| Solution B | 80% acetonitrile aqueous solution containing 0.1% TFA |

**Table 1**

| Retention Time | Composition of Eluent | Flow Rate (ml/min) |
|---|---|---|
| 0-5 mm | Solution A, 100% | 0.15 |
| 5-30 mm | Solution A, 100→0% (linear gradient) | 0.15 |
| 30-35 mm | Solution A, 0% | 0.15 |
| 35-36 mm | Solution A, 0→100% (linear gradient) | 0.15→0.30 |
| 36-44 mm | Solution A, 100% | 0.30 |
| 44-45 mm | Solution A, 100% | 0.30→0.15 |
| | | |
| Detection: | Absorption at 280 nm | |

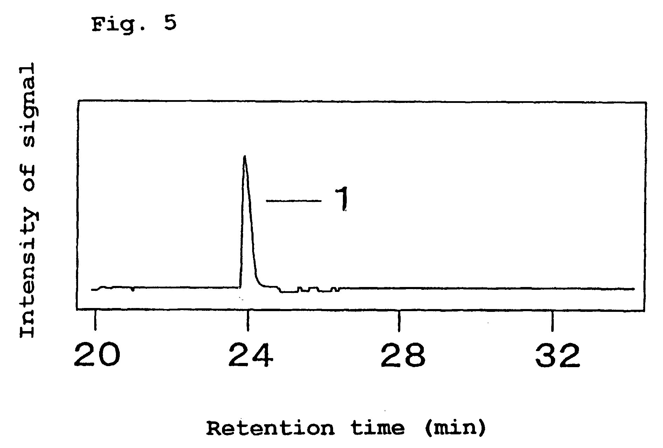

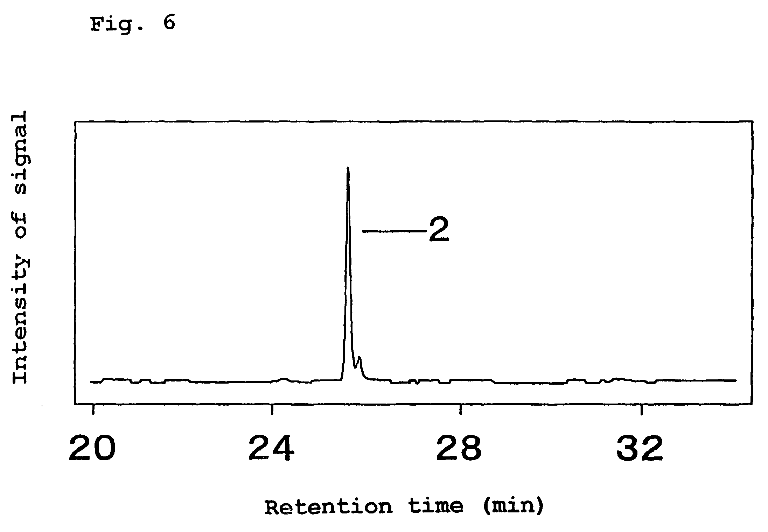

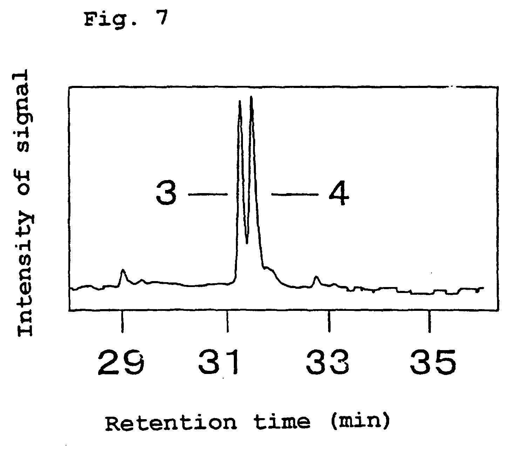

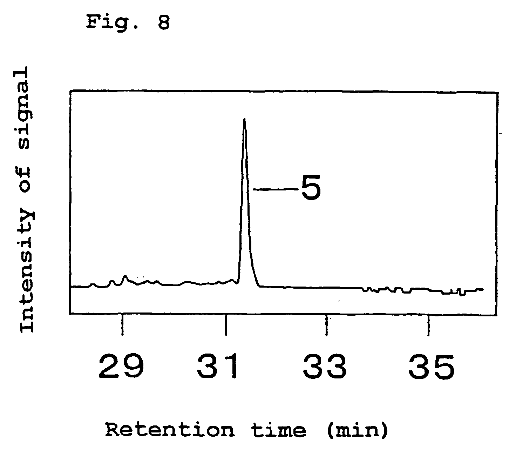

The standard compounds analysed under these conditions are as described below. The results of analysis will be shown in the figures as follows, respectively. Fig. 5 shows an a result of HPLC analysis of alanyltryptophan (Ala-Trp). In the figure, numeral 1 shows a peak corresponding to alanyltryptophan. Fig. 6 shows a result of HPLC analysis analysis of N-acetylalanyltryptophan (Ac-Ala-Trp). In the figure, numeral 2 shows a peak corresponding to N-acetylalanyltryptophan. Fig. 7 shows a result of HPLC analysis analysis of N-acetylalanyl-tryptophanyl-methionyl-arginyl-phenylalanine (Ac-Ala-Trp-Met-Arg-Phe). In the figure, numeral 3 shows a peak corresponding to N-acetylalanyltryptophanylmethionylarginylphenylalanine and numeral 4 shows a peak corresponding to N-acetylalanyltryptophanylmethionylarginyl-phenylalanine. Fig. 8 shows a result of HPLC analysis analysis of N-acetylalanyl-tryptophanyl-methionyl-arginyl-phenylalanyl-alanine (Ac-Ala-Trp-Met-Arg-Phe-Ala). In the figure, numeral 5 shows a peak corresponding to N-acetylalanyltryptophanylmethionylarginylphenylalanylalanine.

In Fig. 7, two peaks corresponding to N-acetylalanyltryptophanyl-methionyl-arginyl-phenylalanine are observed. They are isomers.

### Example 4

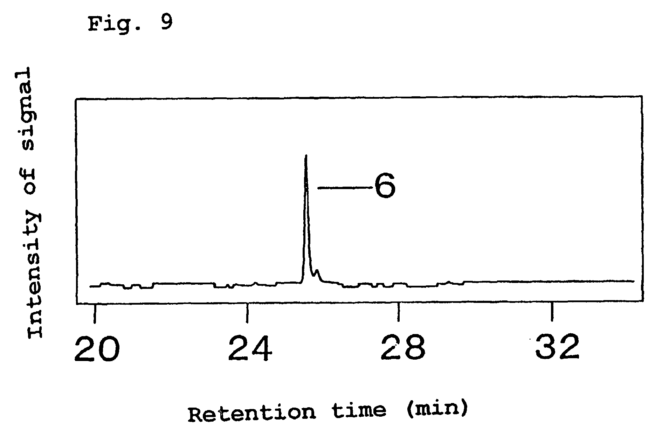

Here, generation of oxazolone under the conditions for the operation of the first process is described. As the sample, alanyltryptophan (Ala - Trp) was used. The oxazolone was detected using the cleavage reaction easily caused by water. Detection was carried out by HPLC analysis. Fig. 9 shows a result of HPLC analysis analysis of the reaction product obtained by allowing the sample obtained in the first process to react with water. The conditions for HPLC analysis analysis are as described in Example 3.

The procedure of preparing the sample for HPLC analysis is as follows.
(1) Evaporate the sample under reduced pressure to dryness.
(2) Dissolve the residue in 0.1% trifluoroacetic acid (referred to as TFA)

Compared with Fig. 7, N-acetylalanyltryptophan (Ac-Ala-Trp) 6 was detected in Fig. 9, this indicates generation of oxazolone in the first process. Here, alanyltryptophan used as the sample was not detected.

### Example 5

Here, cleavage of oxazolone under the conditions for the operation of the second process is described. As the sample, N-acetylalanyltryptophanylmethionylarginylphenylalanylalanine (Ac-Ala-Trp-Met-Arg-Phe-Ala) was used. Detection was carried out by HPLC analysis under the same conditions as in Example 4. The procedure of preparing the sample for HPLC analysis was as described in Example 4.

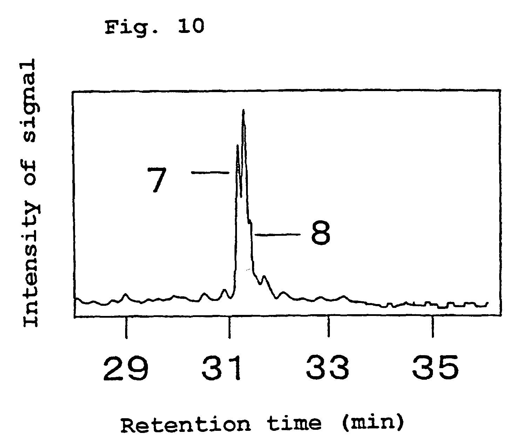

Fig. 10 shows a result of analysing the reaction product obtained in the second process described in Example 2. Compared with Fig. 7, N-acetylalanyltryptophanylmethionylarginylphenylalanine (Ac-AlaTrp-Met-Arg-Phe), denoted by reference numerals 7 and 8, which is a product lacking the C-terminal amino acid, alanine, of N- acetylalanyltryptophanylmethionylarginylphenylalanylalanine (Ac-Ala-Trp-Met-Arg-Phe-Ala) used as the sample was detected in Fig. 10. This indicates cleavage of the oxazolone ring under the conditions for the second process, liberation of the C-terminal amino acid, and generation of the peptide lacking the C-terminal amino acid (having acetylated amino terminal) . This C-terminal amino acid is identified to determine the C-terminal of the peptide.

Then, a combination of the operations consisting of the first and second processes using the peptide lacking the C-terminal amino acid (having acetylated amino terminal) and the operation of isolating and identifying the amino acid obtained is repeatedly carried out to analyse the amino acid sequence of the protein or the peptide used from the C-terminus.

Accordingly, the results of Example 1 to 5 are summarised as follows.

In the present invention, in order to analyse the amino acid sequence from the C-terminus, a combination of the operations consisting of the following first and second processes and the operation of isolating and identifying the amino acid obtained was repeatedly carried out.

### First Process

A protein or a peptide is allowed to react with an acid anhydride, the amino terminal of the protein or the peptide is protected, and the amino acid residue at the C-terminus is modified to generate oxazolone.

### Second Process

The protein or the peptide generated, whose carboxy terminal is oxazolone, is allowed to react with an acid and alcohol to liberate the carboxy-terminal amino acid.

Hereinafter another procedure for carrying out the present invention will be further described.

### Example 6

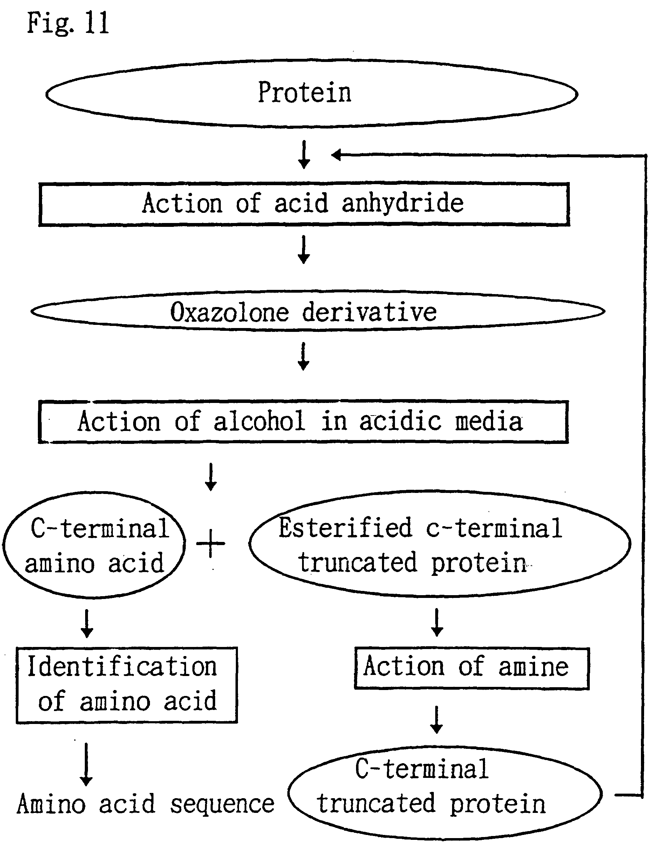

Fig. 11 shows a flow chart illustrating the present invention.

### First Process

A protein or a peptide is allowed to react with an anhydride of an organic acid represented by the general formula;

CH₃- (CH₂) m-CO-O-CO- (CH₂) n-CH₃

(m and n is an integer of 0 or above, respectively) to give a protein or a peptide whose amino group is modified and C-terminal amino acid is converted into oxazolone.

### Second Process

Then, the protein or the peptide, whose amino group is modified and C-terminal amino acid is converted into oxazolone is allowed to react with an alcohol in the presence of acid to give a mixture of a protein or a peptide newly generated whose amino group is modified, the C-terminal amino acid is lacking, and the carboxy group of the C-terminal amino acid is esterified and the original C-terminal amino acid.

### Third Process

The mixture of the protein or the peptide newly generated, whose amino group is modified, C-terminal amino acid is lacking, and carboxy group of the C-terminal amino acid is esterified, and the amino acid is allowed to react with an aqueous solution of an amine, for instance an amine represented by a general formula; NR1R2R3, to give a mixture of a protein or a peptide of which the amino group is modified and the C-terminal amino acid is lacking and the amino acid.

The amino acid obtained in the first to third processes described above is isolated and identified to determine the C-terminal amino acid of the protein or the peptide.

Then a series of the operations of the first to third processes using the protein or the peptide, whose amino group is modified and the C-terminal amino acid is lacking, and the operation of isolating and identifying the amino acid obtained are repeatedly carried out to analyse the amino acid sequence of the protein or the peptide from the C-terminus.

### Example 7

Here, an example of the operation procedure of the present invention is described which analyses an amino acid sequence of a protein or a peptide from a C-terminus thereof by repeating a series of the operations of the first to third processes using a protein or a peptide and an operation of isolating and identifying the amino acid from the mixture obtained in the second process.

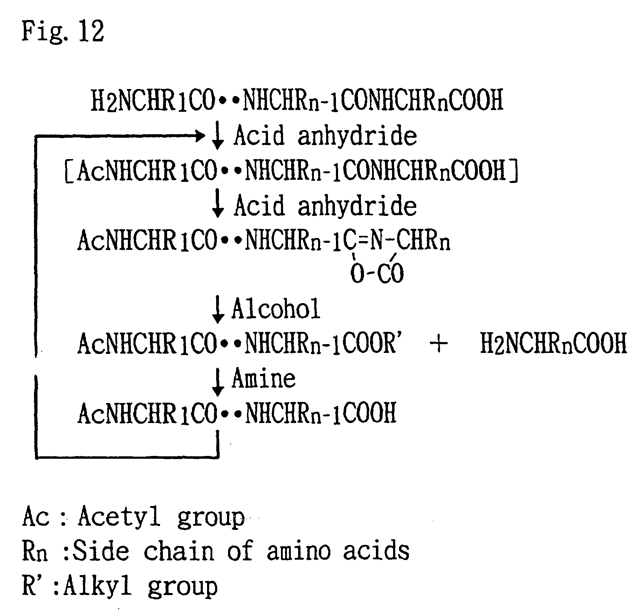

Fig. 12 shows an example of a reaction scheme illustrating an experimental procedure of the present invention.

### First Process

A sample is allowed to react with acetic anhydride. An example of the reaction conditions is as follows.

| Reaction conditions | |
|---|---|
| Concentration of acetic anhydride | 20% |
| (acetonitrile solution, acetic acid added to adjust to 1% of the concentration) | |
| Reaction temperature | 60°C |
| Reaction period | 30 minutes |

After this reaction, the reaction mixture is evaporated under reduced pressure to dryness to remove the reagent and solvent.

### Second Process

The sample obtained thereby is allowed to react with methanol in the presence of pentafluoropropionic acid (CF3 - CF2-COOH: hereinafter referred to as PFPA) 1. An example of the reaction conditions is as follows.

| Reaction conditions | |
|---|---|
| Concentration of PFPA | 10% (methanol solution) |
| Reaction temperature | 60°C |
| Reaction period | 30 minutes |

After this reaction, the reaction mixture is evaporated under reduced pressure to dryness to remove the reagent and solvent.

### Third Process

The sample obtained thereby is allowed to react with 2-dimethylaminoethanol (hereinafter referred to as DMAE). The reaction conditions are as follows.

| Reaction conditions | |
|---|---|
| Concentration of DMAE | 10% (aqueous solution) |
| Reaction temperature | 60°C |
| Reaction period | 30 minutes |

In a series of these operations, after the second process, the amino acid is isolated from the sample obtained and identified.

Then, the combination of a series of the procedures of the first to third processes using the protein or the peptide, whose amino group is modified and the C-terminal amino acid is lacking, and the operation of isolating and identifying the amino acid obtained in the second process is repeatedly carried out to analyse the amino acid sequence of the protein or the peptide from the C-terminus.

### Example 8

Here, an example of the operation procedure of the present invention is described which analyses an amino acid sequence of a protein or a peptide from a C-terminus thereof by repeating a combination of a series of the operations of the first to third processes using a protein or a peptide and an operation of isolating and identifying the amino acid from the mixture obtained in the third process.

A series of the operations of the first to third processes is the same as described in Example 7. In a series of these operations, after the third process, the amino acid is isolated from the sample obtained and identified.

Then, a series of the procedures of the first to third processes using the protein or the peptide, whose amino group is modified and the C-terminal amino acid is lacking, and the operation of isolating and identifying the amino acid obtained in the third process are repeatedly carried out to analyse the amino acid sequence of the protein or the peptide from the C-terminus.

### Example 9

Here, the conditions for HPLC analysis analysis describing the present invention and the results of analysing standard compounds by HPLC are described.

The conditions for the HPLC analysis were as follows.

| Analysis conditions | |
|---|---|
| Column | Shiseido C18 MICROBORE |
| Elution | Gradient elution with the following solution A and B |
| Solution A | 0.1% TFA aqueous solition |
| Solution B0.1% TFA aqueous solition | 80% acetonitrile aqueous solution containing 0.1% TFA |

**Table 2**

| Retention Time | Composition of Eluent | Flow Rate (ml/min) |
|---|---|---|
| 0-5 mm | Solution A, 100% | 0.15 |
| 5-30 mm | Solution A, 100→0% (linear gradient) | 0.15 |
| 30-35 mm | Solution A, 0% | 0.15 |
| 35-36 mm | Solution A, 0→100% (linear gradient) | 0.15→.30 |
| 36-44 mm | Solution A, 100% | 0.30 |
| 44-45 mm | Solution A, 100% | 0.30→0.15 |

**Table 2**

| Retention Time | Composition of Eluent | Flow Rate (ml/min) |
|---|---|---|
| 0-5 mm | Solution A, 100% | 0.15 |
| 5-30 mm | Solution A, 100→0% (linear gradient) | 0.15 |
| 30-35 mm | Solution A, 0% | 0.15 |
| 35-36 mm | Solution A, 0→100% (linear gradient) | 0. 15→. 30 |
| 36-44 mm | Solution A, 100% | 0.30 |
| 44-45 mm | Solution A, 100% | 0. 30→0.15 |
| | | |
| Detection: | Absorption at 280 nm | |

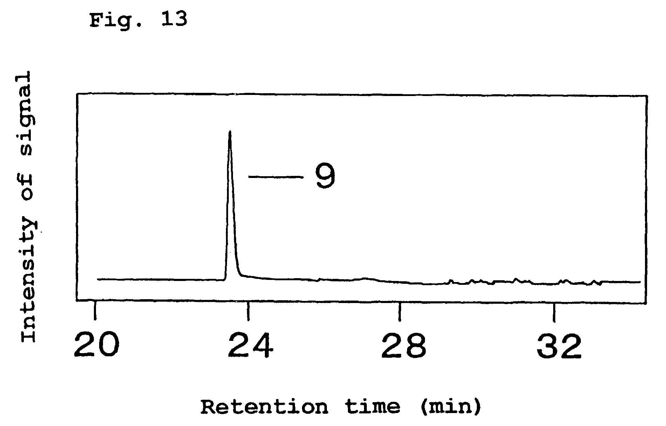

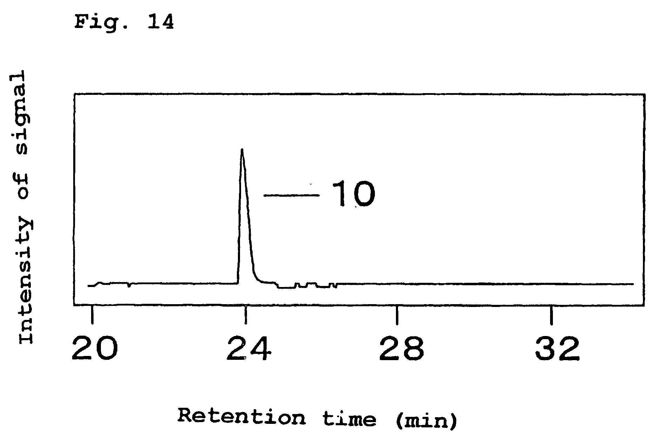

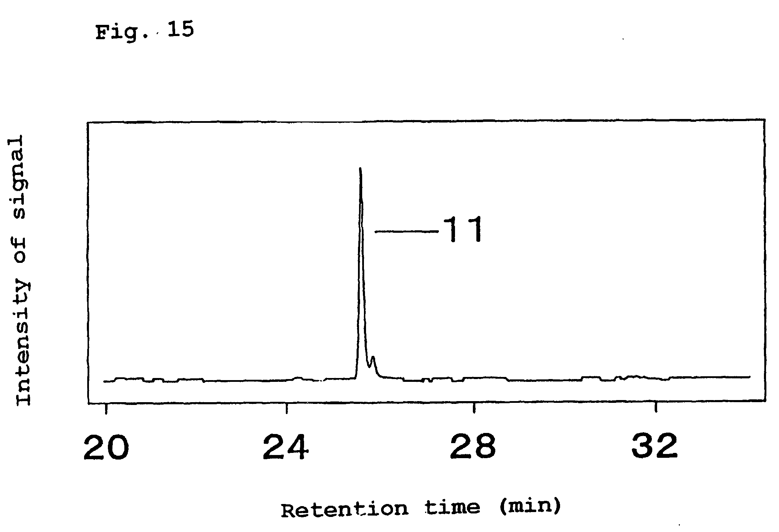

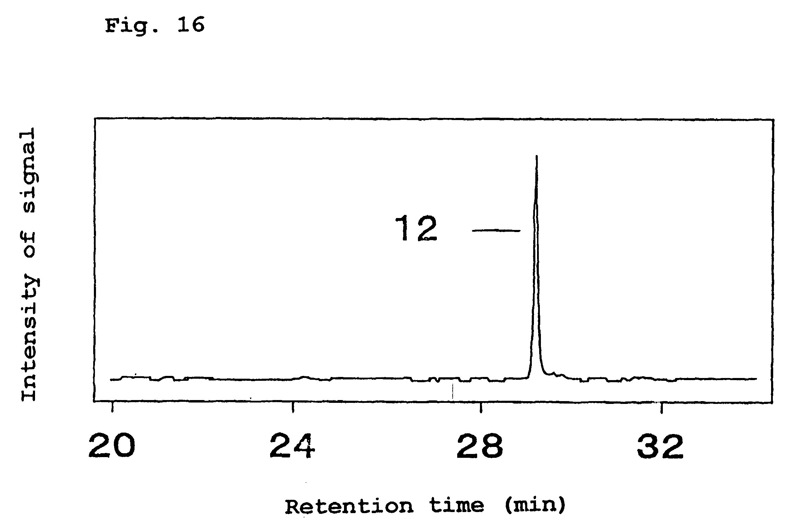

The standard compounds analysed under these conditions are as described below. The results of analysis will be shown in the figures as follows, respectively. Fig. 13 shows the result of HPLC analysis analysis of tryptophan (Trp) . In the figure, numeral 9 shows a peak corresponding to tryptophan. Fig. 14 shows that of alanyltryptophan (Ala-Trp). In the figure, numeral 10 shows a peak corresponding to alanyltryptophan. Fig. 15 shows that of N-acetylalanyltryptophan (Ac-Ala-Trp). In the figure, numeral 11 shows a peak corresponding to N-acetylalanyltryptophan. Fig. 16 shows that of the ethyl ester of N-acetylalanyltryptophan (Ac-Ala-Trp-OEt). In the figure, numeral 12 shows a peak corresponding to the ethylester of N-acetylalanyltryptophan.

### Example 10

Here, generation of oxazolone under the conditions in the operation of the first process is described. As the sample, alanyltryptophan (Ala-Trp) was used. The oxazolone was detected using the cleavage reaction easily caused by water.
Detection was carried out by HPLC analysis. Fig. 9 shows the result of HPLC analysis analysis of the reaction product obtained by allowing the sample obtained in the first process to react with water. The conditions for HPLC analysis analysis are as described in Example 9.

The procedure of preparing the sample for HPLC analysis is as follows.
(1) Evaporate the sample under reduced pressure to dryness.
(2) Dissolve the residue in 0.1% TFA

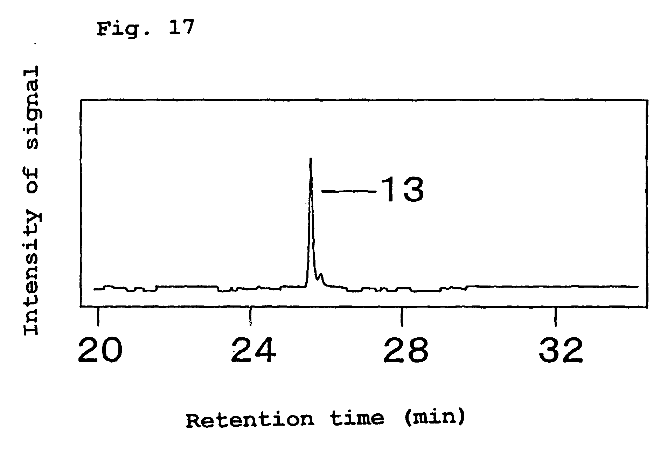

Compared with Fig. 15, N-acetylalanyltryptophan (Ac-Ala -Trp) denoted by reference numeral 13 was detected in Fig. 17, this indicates generation of oxazolone in the first process. Here, alanyltryptophan used as the sample was not detected.

### Example 11

Here, cleavage of oxazolone under the conditions in the operation of the second process is described. As the sample, alanyltryptophan was used in the same manner as in Example 10. Detection was carried out by HPLC analysis under the same conditions as in Example 9. The procedure of preparing the sample for HPLC analysis was as described in Example 5.

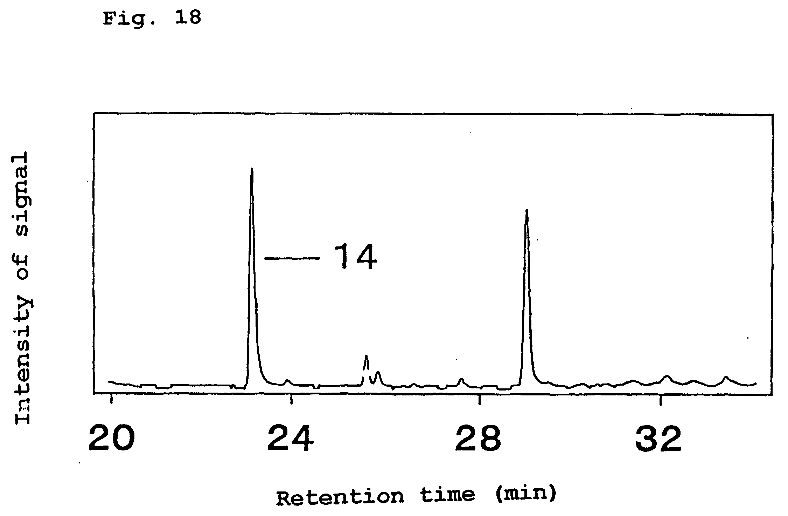

Fig. 10 shows the result of analysing the reaction product obtained in the first and second processes described in Example 7. Compared with Fig. 13, tryptophan (reference numeral 14) was detected in Fig. 18 to indicate cleavage of the oxazolone ring under the conditions in the second process and liberation of the C-terminal amino acid.

### Example 12

In Example 11, the oxazolone was allowed to react with an alcohol solution of PFPA to liberate the carboxy-terminal amino acid. By the action of this alcohol (alcoholysis), the carboxyl group newly generated at the C-terminus is esterified in concurrence with the liberation of the amino acid. In order to continue the amino acid sequencing of the protein or the peptide from the C-terminus, the ester requires to be hydrolyzed to give a carboxyl group.

Here, hydrolysis of the ester under the conditions in the operation of the third process is described. As the sample, the ethyl ester of N-acetylalanyltryptophan (Ac-Ala-Trp-QEt) was used. Detection was carried out by HPLC analysis under the same conditions as in Example 9.

The procedure of preparing the sample for HPLC analysis was as described in Example 5.

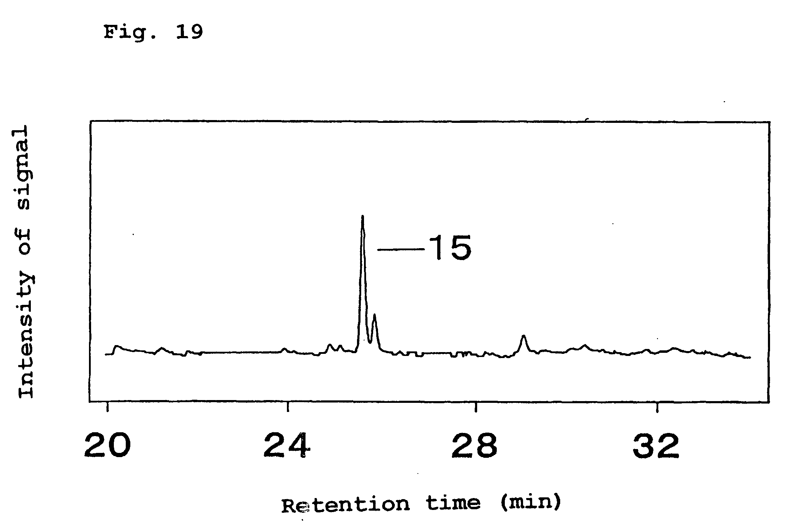

Fig. 11 shows the result of analysing the reaction product obtained in the third process described in Example 7. Compared with Fig. 15, N-acetylalanyltryptophan (Ac-Ala-Trp) (reference numeral 15) was detected in Fig. 19 to indicate hydrolysis of the ester by the action of the aqueous solution of amine under the conditions in the third process of Example 7.

### Example 13

In the subsequent examples, the present invention will be described using mass spectrometry.

This example shows the conditions of mass spectrometric analysis and the procedure for sample preparation.

| Conditions for mass spectrometric analysis | |
|---|---|
| Analyzer: | Double focusing mass spectrometer HX-110 (Nihon Denshi) |
| Analysis conditions: | Accelerating potential 10kV |
| Resolution | 1,000 |
| Ion source | FAB(fast-atom bombardment method) |
| Ionization gas | Xe |
| Ion mode | cationic |
| FAB gun accelerating potential | 6kV |
| Detector | MULTIPLIER |
| Load potential | -20kv |
| Data processing system | DA5000 |
| Matrix | |
| glycerol: thioglycerol :m-nitrobenzyl alcohol =1:1:1 | |

### Procedure for sample preparation

(1) Evaporate a sample under reduced pressure to dryness.
(2) Dissolve the residue in 67% acetic acid (or dimethylformamide) aqueous solution.
(3) Place the matrix of 1µl on the target.
(4) Place the sample solution of 1µl on the target and allow them to mix.
(5) Introduce the mixture into the ion source.

### Example 14

Here, an investigation of the reaction conditions for generation of oxazolone in the first process using mass spectrometry is described. As the sample, a pentapeptide of Sequence No.1, leucyltryptophanylmethionylarginylphenylalanine (Leu-Trp-MetArg-Phe), was used. In this example, the second process was successively carried out after the first process.

The reaction conditions in the first process are the same as in Example 2 as follows.

| Reaction conditions | |
|---|---|
| Concentration of acetic anhydride | 20% |
| (acetonitrile solution, acetic acid added to adjust to 1% of the concentration) | |
| Reaction temperature | 60°C |
| Reaction period | 30 minutes |

The reaction conditions in the second process were as follows. Heptafluorobutyric acid (CF3-CF2-CF2-COOH; hereinafter referred to as HFBA) and ethanol were used as the acid and the alcohol, respectively.

| Reaction conditions | |
|---|---|
| Concentration of HFBA | 5 % (ethanol solution) |
| Reaction temperature | 60°C |
| Reaction period | 30 minutes |

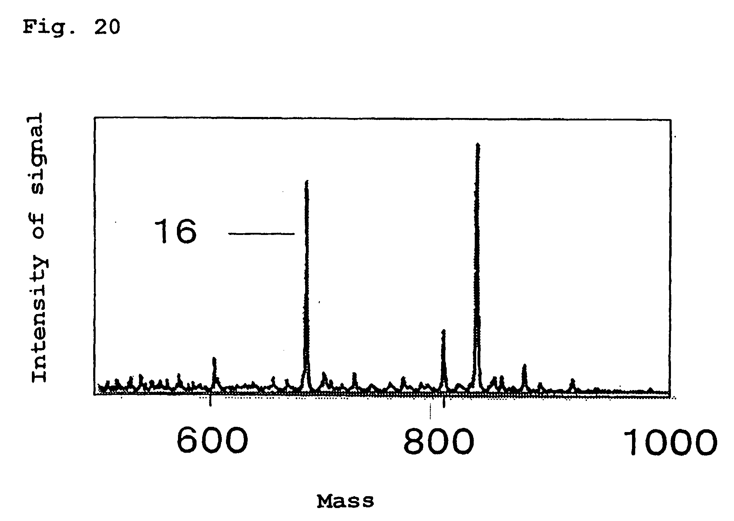

Fig. 20 shows a result of mass spectrometric analysis of the sample obtained. Here, a molecular ion (reference numeral 16) corresponding to the reaction product whose amino terminal was acetylated, C-terminal amino acid was lacking, and carboxyl group at the C-terminus was esterified with an ethyl group (the ethyl ester of acetylleucyltriptophanylmethionylarginine molecular weight of 675; hereinafter referred to as Ac-LWMR-OEt) was detected to indicate generation of oxazolone in the first process and liberation of the C-terminal amino acid in the second process.

### Example 15

Here, another example of the reaction conditions in the first process is described. As the sample, the pentapeptide of Sequence No.1 was used in the same manner as in Example 14. The procedures are the same as in Example 14. The reaction conditions in the first process were as follows.

| Reaction conditions | |
|---|---|
| Concentration of acetic anhydride | 20% |
| (acetonitrile solution, without addition of acetic acid) | |
| Reaction temperature | 60°C |
| Reaction period | 30 minutes |

The reaction conditions in the second process are the same as in Example 13.

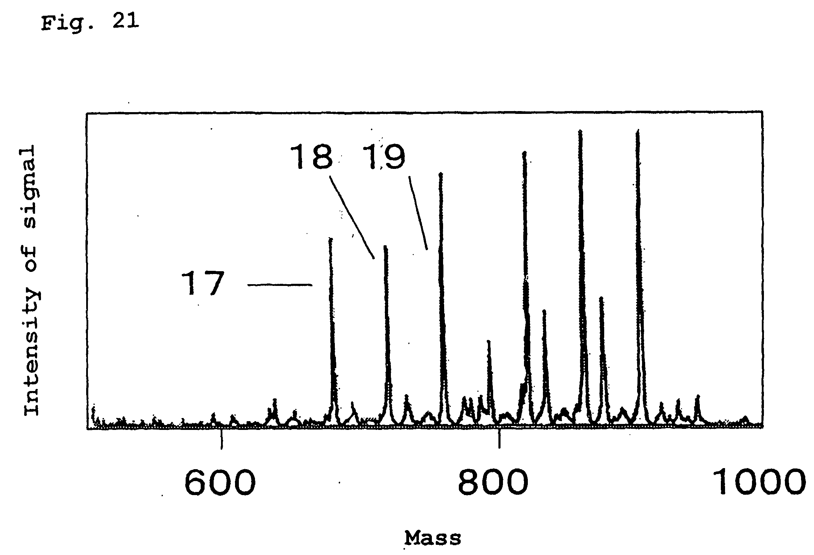

Fig. 21 shows a result of mass spectrometric analysis of the sample obtained. Under these conditions, also, molecular ion corresponding to Ac-LWMR-OEt was detected (reference numeral 17) to indicate generation of oxazolone in the first process. Simultaneously, molecular ion having 718 and 761 of molecular weight were detected (reference numerals 18 and 19, respectively), to indicate generation of chemical species which were further acetylated reaction products, whose amino terminal was acetylated, the C-terminal amino acid was lacking, and the carboxyl group at the C-terminus was esterified with an ethyl group.

### Example 16

Here, another example of the reaction conditions in the first process is described. As the sample, the pentapeptide of Sequence No. 1 was used in the same manner as in Example 9. The procedures followed in Example 14. The reaction conditions in the first process were as follows.

| Reaction conditions | |
|---|---|
| Concentration of acetic anhydride | 20% |
| (acetonitrile solution, acetic acid added to adjust to 2% of the concentration) | |
| Reaction temperature | 60°C |
| Reaction period | 30 minutes |

The reaction conditions in the second process are the same as in Example 16.

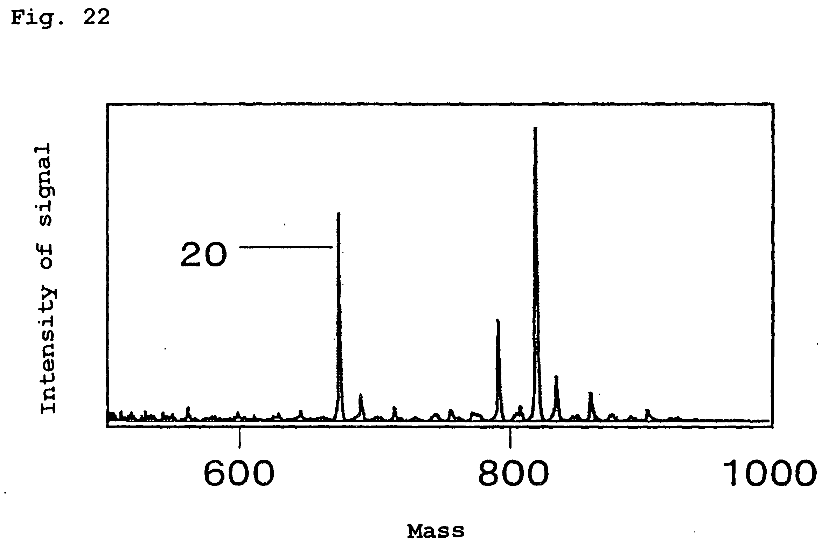

Fig. 22 shows a result of mass spectrometric analysis of the sample obtained. Under these conditions, also, molecular ion corresponding to Ac-LWMR-OEt (reference numeral 20) was detected to indicate generation of oxazolone in the first process.

### Example 17

Here, another example of the reaction conditions in the first process is described. As the sample, the pentapeptide of Sequence No.1 was used in the same manner as in Example 9. The procedures followed in Example 14. The reaction conditions in the first process were as follows.

| Reaction conditions | |
|---|---|
| Concentration of acetic anhydride | 5% (acetonitrile solution, acetic acid added to adjust to 1% of the concentration) |
| Reaction temperature | 60°C |
| Reaction period | 30 minutes |

The reaction conditions in the second process are the same as in Example 14.

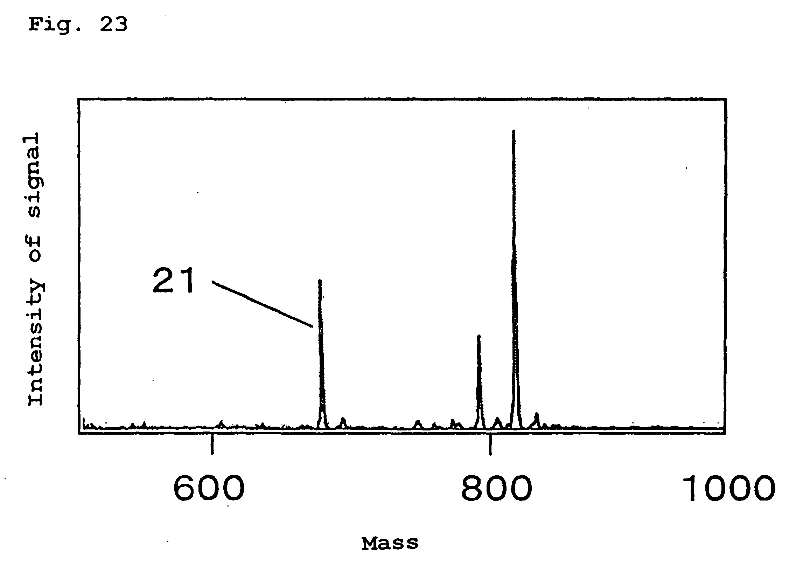

Fig. 23 shows a result of mass spectrometric analysis of the sample obtained. Under these conditions, also, molecular ion corresponding to Ac-LWMR-OEt (reference numeral 21) was detected to indicate generation of oxazolone in the first process.

### Example 18

Here, an investigation of the reaction conditions for liberation of the C-terminal amino acid in the second process is described. As the sample, a pentapeptide of Sequence No.1 was used. In the subsequent examples, also, the second process was successively carried out after the first process.

The reaction conditions in the first process are the same as in Example 14 as follows.

| Reaction conditions | |
|---|---|
| Concentration of acetic anhydride | 20% (acetonitrile solution, acetic acid added to adjust to 1% of the concentration) |
| Reaction temperature | 60°C |
| Reaction period | 30 minutes |

The reaction conditions in the second process were changed as follows.

| Reaction conditions | |
|---|---|
| Concentration of HFBA | 2% (ethanol solution) |
| Reaction temperature | 60°C |
| Reaction period | 30 minutes |

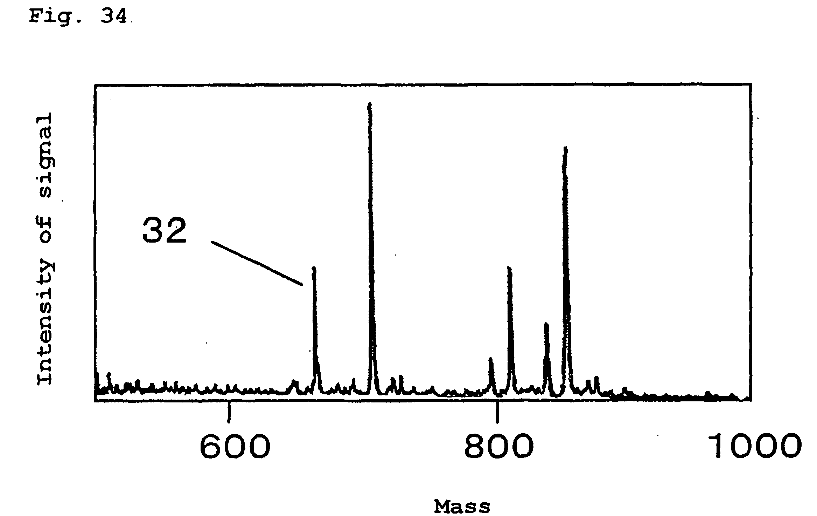

Fig. 34 shows a result of mass spectrometric analysis of the sample obtained. Here, a molecular ion corresponding to Ac-LWMR-OEt (reference numeral 22) was detected to indicate generation of oxazolone in the first process and liberation of the C-terminal amino acid in the second process.

### Example 19

Here, the reaction conditions in the second process were changed as follows.

| Reaction conditions | |
|---|---|
| Concentration of HFBA | 5% (ethanol solution) |
| Reaction temperature | 60°C |
| Reaction period | 10 minutes |

The reaction conditions in the first process followed in Example 9.

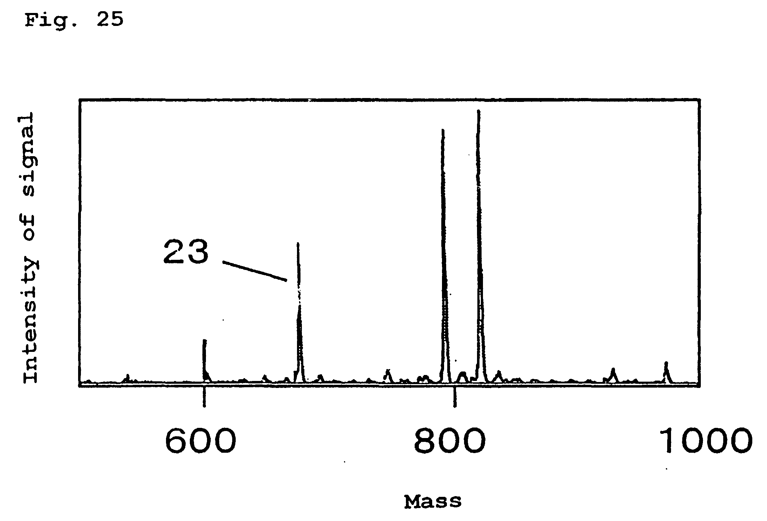

Fig. 25 shows a result of mass spectrometric analysis of the sample obtained. Under these conditions, also, a molecular ion corresponding to Ac-LWMR-OEt (reference numeral 23) was detected to indicate generation of oxazolone in the first process and liberation of the C-terminal amino acid in the second process.

### Example 20

Here, the reaction conditions in the second process were changed as follows.

| Reaction conditions | |
|---|---|
| Concentration of HFBA | 5% (ethanol solution) |
| Reaction temperature | room temperature |
| Reaction period | 30 minutes |

The reaction conditions in the first process are the same as in Example 14.

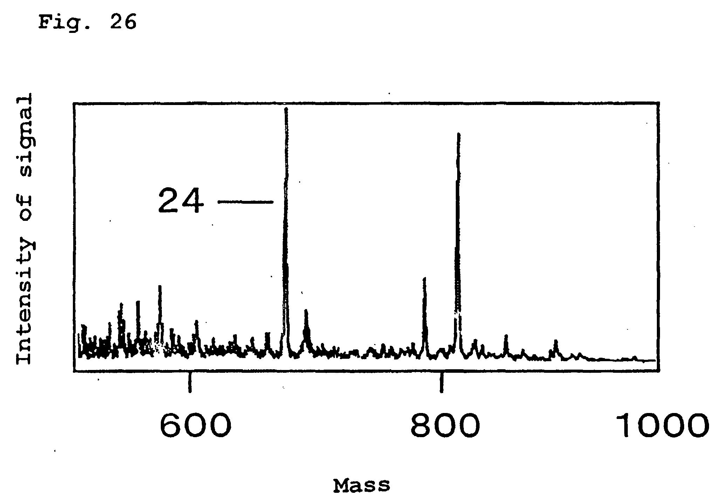

Fig. 26 shows the result of mass spectrometric analysis of the sample obtained. Under these conditions, also, a molecular ion corresponding to Ac-LWMR-OEt (reference numeral 24) was detected to indicate generation of oxazolone in the first process and liberation of the C-terminal amino acid in the second process.

### Example 21

Here, the reaction conditions in the second process were changed as follows.

| Reaction conditions | |
|---|---|
| Concentration of HFBA | 5% (ethanol solution) |
| Reaction temperature | 5°C |
| Reaction period | 30 minutes |

The reaction conditions in the first process are the same as in Example 14.

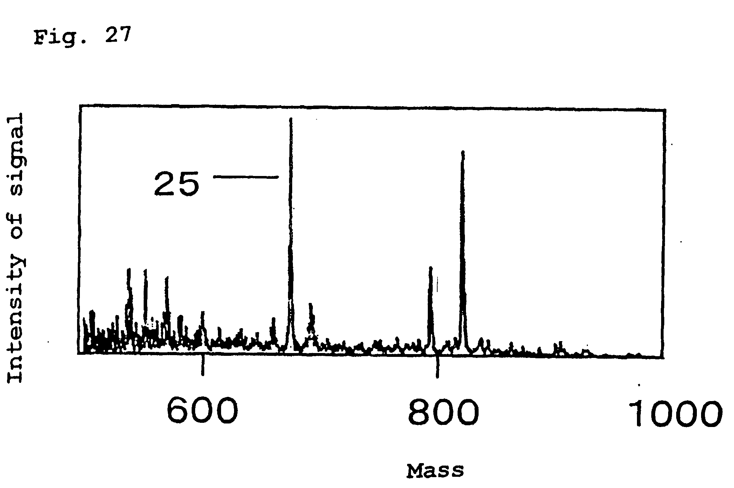

Fig. 27 shows the result of mass spectrometric analysis of the sample obtained. Under these conditions, also, a molecular ion corresponding to Ac-LWMR-OEt (reference numeral 25) was detected to indicate generation of oxazolone in the first process and liberation of the C-terminal amino acid in the second process.

### Example 22

Here, the reaction conditions in the second process were changed as follows.

| Reaction conditions | |
|---|---|
| Concentration of HFBA | 5% (ethanol solution) |
| Reaction temperature | 100°C |
| Reaction period | 15 minutes |

The reaction conditions in the first process followed in Example 14.

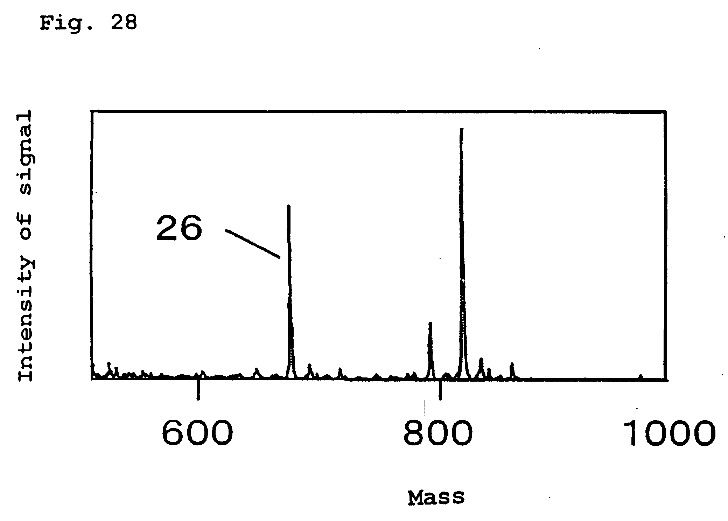

Fig. 28 shows a result of mass spectrometric analysis of the sample obtained. Under these conditions, also, a molecular ion corresponding to Ac-LWMR-OEt (reference numeral 26) was detected to indicate generation of oxazolone in the first process and liberation of the C-terminal amino acid in the second process.

### Example 23

Here, the reaction conditions in the second process were changed as follows.

| Reaction conditions | |
|---|---|
| Concentration of HFBA | 5 % (methanol solution) |
| Reaction temperature | 60°C |
| Reaction period | 30 minutes |

The reaction conditions in the first process are the same as in Example 14.

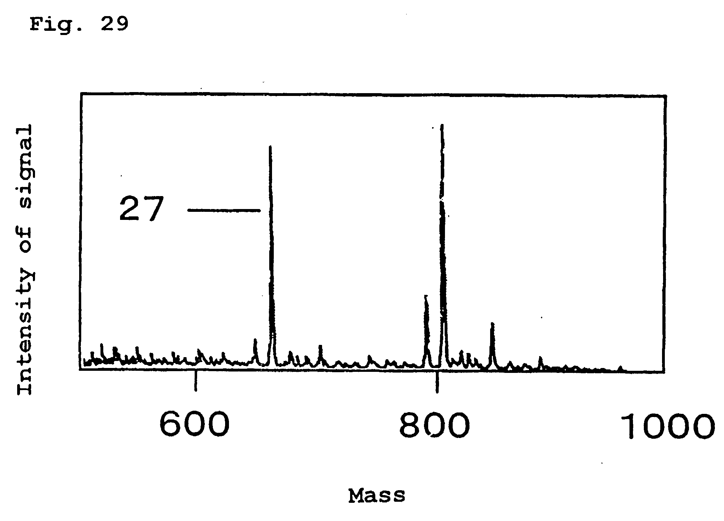

Fig. 29 shows a result of mass spectrometric analysis of the sample obtained. Under these conditions, a molecular ion (corresponding to the reaction product, whose amino terminus was acetylated, C-terminal amino acid was lacking, and carboxyl group at the C-terminus was esterified with a methyl group (the methyl ester of acetylleucyltriptophanylmethionylarginine, molecular weight of 661; hereinafter referred to as Ac-LWMR-OMe) (reference numeral 27) was detected to indicate generation of oxazolone in the first process and liberation of the C-terminal amino acid in the second process.

### Example 24

Here, the reaction conditions in the second process were changed as follows.

| Reaction conditions | |
|---|---|
| Concentration of HFBA | 5% (methanol solution) |
| Reaction temperature | 5°C |
| Reaction period | 30 minutes |

The reaction conditions in the first process are the same as in Example 14.

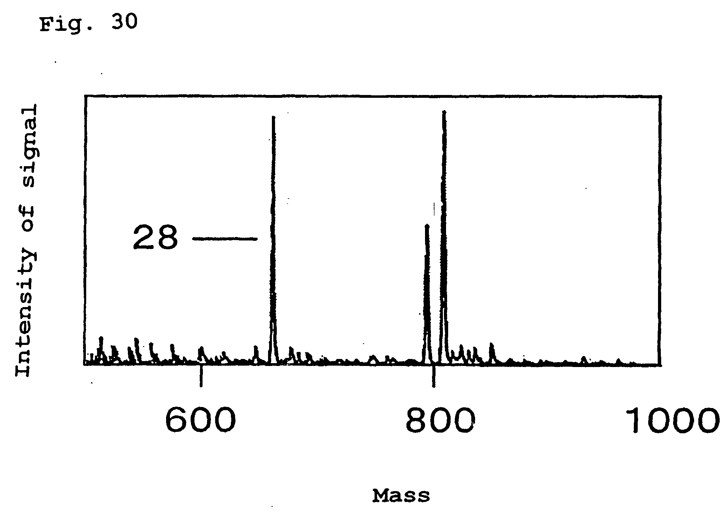

Fig. 30 shows a result of mass spectrometric analysis of the sample obtained. Under these conditions, also, a molecular ion corresponding to Ac-LWMR-OMe (reference numeral 28) was detected to indicate generation of oxazolone in the first process and liberation of the C-terminal amino acid in the second process.

### Example 25

Here, the reaction conditions in the second process were changed as follows.

| Reaction conditions | |
|---|---|
| Concentration of PFPA | 5% (ethanol solution) |
| Reaction temperature | 60°C |
| Reaction period | 30 minutes |

The reaction conditions in the first process are the same as in Example 14.

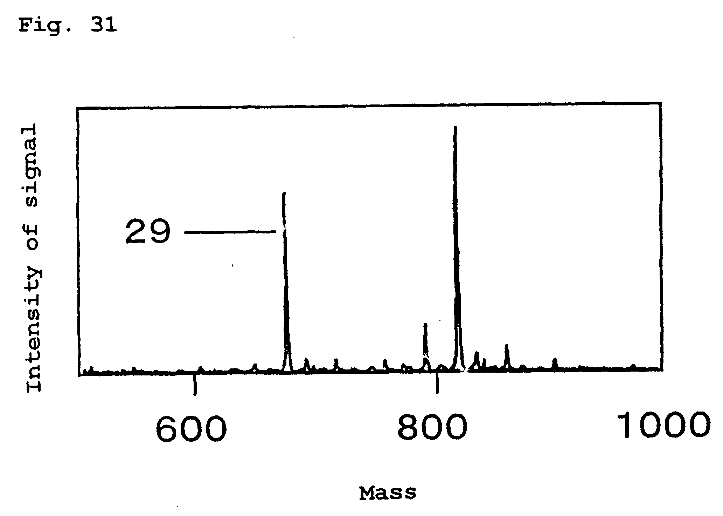

Fig. 31 shows a result of mass spectrometric analysis of the sample obtained. Under these conditions, a molecular ion corresponding to Ac-LWMR-OEt (reference numeral 29) was detected to indicate generation of oxazolone in the first process and liberation of the C-terminal amino acid in the second process.

### Example 26

Here, the reaction conditions in the second process were changed as follows.

| Reaction conditions | |
|---|---|
| Concentration of PFPA | 20% (ethanol solution) |
| Reaction temperature | 60°C |
| Reaction period | 30 minutes |

The reaction conditions in the first process are the same as in Example 14.

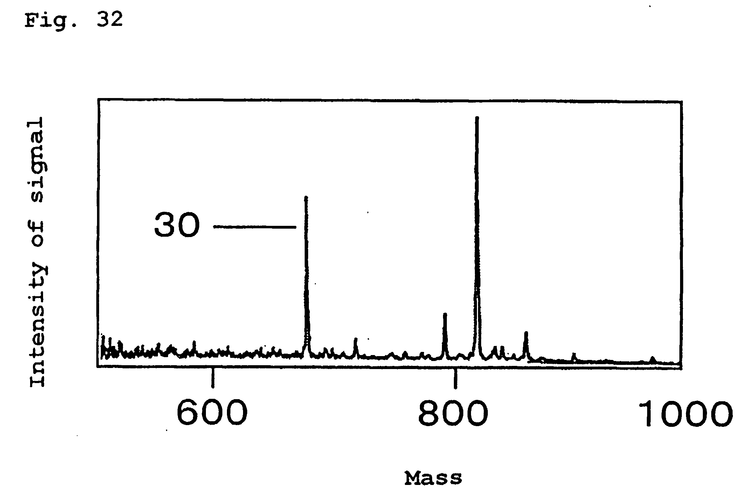

Fig. 32 shows a result of mass spectrometric analysis of the sample obtained. Under these conditions, also, a molecular ion corresponding to Ac-LWMR-OEt (reference numeral 30) was detected to indicate generation of oxazolone in the first process and liberation of the C-terminal amino acid in the second process.

### Example 27

Here, the reaction conditions in the first process were as follows.

| Reaction conditions | |
|---|---|
| Concentration of acetic anhydride | 20% (acetonitrile solution, acetic acid added to adjust to 1 % of the concentration) |
| Reaction temperature | 60°C |
| Reaction period | 10 minutes |

The reaction conditions in the second process were as follows.

| Reaction conditions | |
|---|---|
| Concentration of HFBA | 5% (methanol solution) |
| Reaction temperature | room temperature |
| Reaction period | 30 minutes |

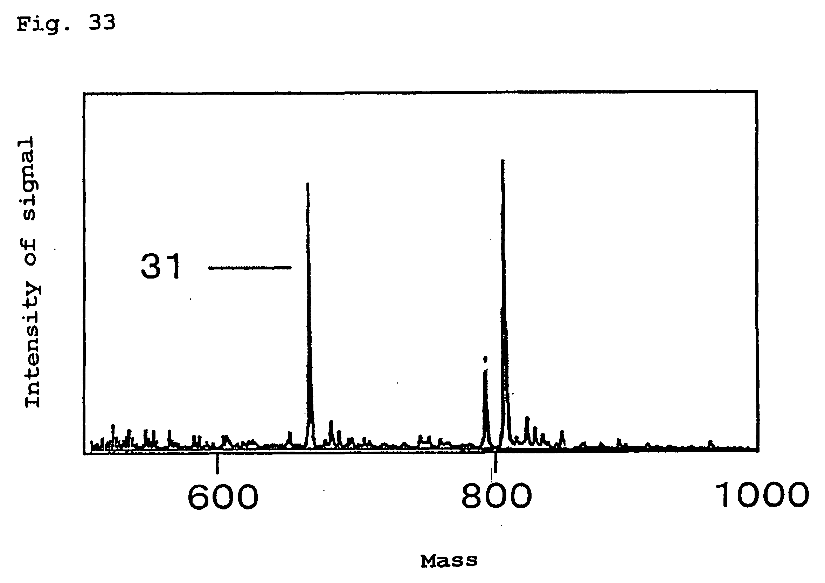

Fig. 33 shows a result of mass spectrometric analysis of the sample obtained. Under these conditions, also, a molecular ion corresponding to Ac-LWMR-OMe (reference numeral 31) was detected to indicate generation of oxazolone in the first process.

### Example 28

Here, the reaction conditions in the first process were as follows.

| Reaction conditions | |
|---|---|
| Concentration of acetic anhydride | 20% (acetonitrile solution, acetic acid added to adjust to 1 % of the concentration) |
| Reaction temperature | 90°C |
| Reaction period | 10 minutes |

The reaction conditions in the second process were as follows.

| Reaction conditions | |
|---|---|
| Concentration of HFBA | 5% (methanol solution) |
| Reaction temperature | room temperature |
| Reaction period | 30 minutes |

Fig. 34 shows a result of mass spectrometric analysis of the sample obtained. Under these conditions, also, a molecular ion corresponding to Ac-LWMR-OMe (reference numeral 32) was detected to indicate generation of oxazolone in the first process and liberation of the C-terminal amino acid in the second process.

### Example 29

Here, the reaction conditions in the first process were as follows.

| Reaction conditions | |
|---|---|
| Concentration of acetic anhydride | 20% (acetonitrile solution, acetic acid added to adjust to 1% of the concentration) |
| Reaction temperature | room temperature |
| Reaction period | 30 minutes |

The reaction conditions in the second process were changed as follows.

| Reaction conditions | |
|---|---|
| Concentration of HFBA | 5% (methanol solution) |
| Reaction temperature | 60°C |
| Reaction period | 30 minutes |

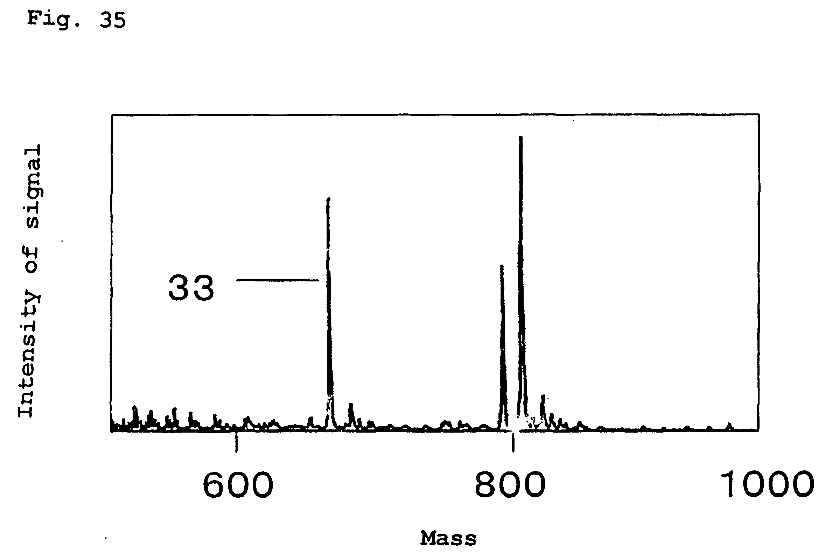

Fig. 35 shows a result of mass spectrometric analysis of the sample obtained. Under these conditions, also, a molecular ion corresponding to Ac-LWMR-OMe (reference numeral 33) was detected to indicate generation of oxazolone in the first process and liberation of the C-terminal amino acid in the second process.

Accordingly, the results of Example 6 to 29 are summarized as follows.

In the present invention, in order to analyse the amino acid sequence from the C-terminus, a combination of a series of the operations consisting of the following first to third processes and the operation of isolating and identifying the amino acid obtained was repeatedly carried out.

### First Process

A protein or a peptide is allowed to react with an acid anhydride, the amino group of the protein or the peptide is protected, and the amino acid residue at the C-terminus is modified to generate oxazolone.

### Second Process

The reaction product in the first process is allowed to react with an alcohol in the presence of acid, the oxazolone contained therein is subjected to alcoholysis (esterification), and the carboxy-terminal amino acid is liberated.

### Third Process

The reaction product in the second process is allowed to react with an amine, the ester contained therein is hydrolyzed.

Hereinafter another procedure for carrying out the present invention will be further described.

### Example 30

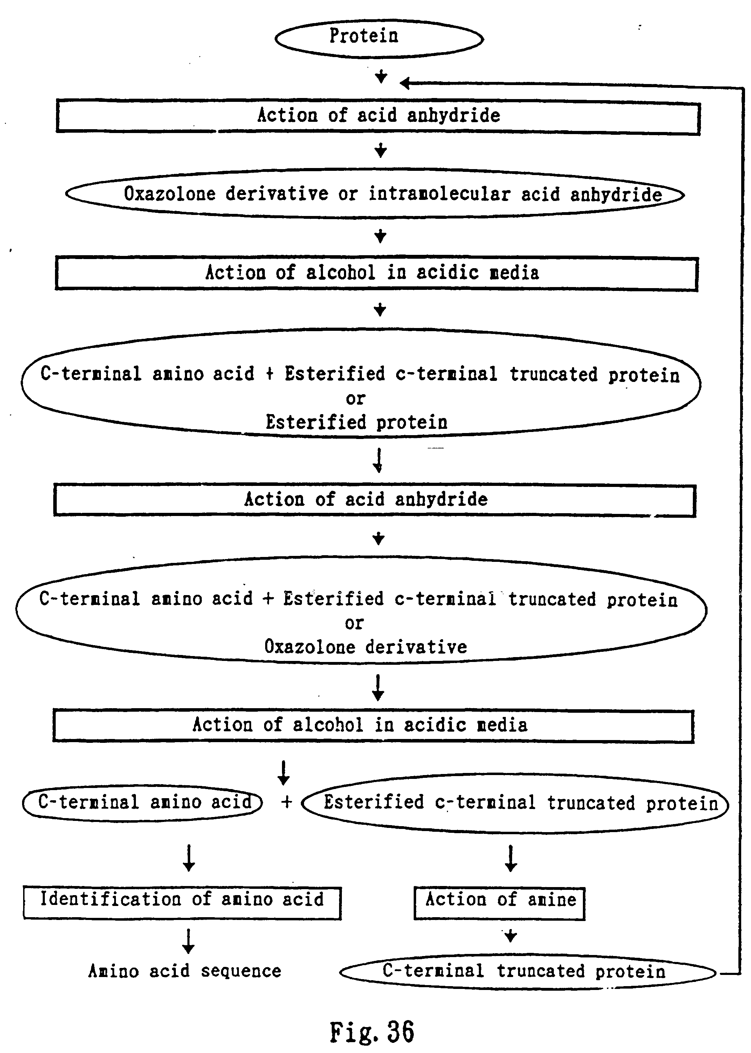

Fig. 36 shows a flow chart illustrating the present invention.

### First Process

A protein or a peptide is allowed to react with an anhydride of an organic acid represented by the general formula;

CH₃- (CH₂) m-CO-O-CO- (CH₂) n-CH₃

(m and n is an integer of 0 or above, respectively) to give a protein or a peptide of which the amino group is modified and C-terminal amino acid is converted into oxazolone or to give a protein or a peptide of which the amino group is acylated and the aspartic acid at the C-terminus is converted into an intramolecular acid anhydride when the C-terminal amino acid is aspartic acid.

### Second Process

Then, the protein or the peptide of which the amino group is modified and C-terminal amino acid is converted into oxazolone or the protein or the peptide of which the amino group is acylated and the aspartic acid at the C-terminus is converted into an intramolecular acid anhydride is allowed to react with an alcohol in the presence of acid to give a mixture of a protein or a peptide newly generated of which the amino group is acylated, the C-terminal amino acid is lacking, and the carboxyl group of the C-terminal amino acid is esterified and the original C-terminal amino acid. At this time, the protein or the peptide of which the amino group is acylated and the aspartic acid at the C-terminus is converted into an intramolecular acid anhydride is converted into a protein or a peptide of which the amino group is acylated and the B-carboxyl group of the aspartic acid at the C-terminus is esterified.

### Third Process

The mixture of a protein or a peptide newly generated, whose amino group is acylated, C-terminal amino acid is lacking, and carboxyl group of the C-terminal amino acid is esterified, and the original C-terminal amino acid, or the protein or the peptide, whose amino group is acylated, and β-carboxyl group of the aspartic acid at the C-terminus is esterified, is allowed to react with an anhydride of an organic acid represented by the general formula; CH₃-(CH₂)ₘ-CO-O-CO-(CH₂)ₙ-CH₃ (m and n is an integer of 0 or above, respectively) . At this time, the protein or the peptide newly generated, whose amino group is modified, C-terminal amino acid is lacking, and carboxyl group of the C-terminal amino acid is esterified, is not affected at all. The original C-terminal amino acid is converted into an acylated amino acid derivative. On the other hand, the protein or the peptide, whose amino group is acylated and the β-carboxyl group of the aspartic acid at the C-terminus is esterified, is converted into a protein or a peptide, whose amino group is acylated, α-carboxyl group of the aspartic acid at the C-terminus is converted into oxazolone, and β-carboxyl group is esterified.

### Fourth Process

Furthermore, the mixture of a protein or a peptide newly generated, whose amino group is acylated, C-terminal amino acid is lacking, and carboxyl group of the C-terminal amino acid is esterified, and the amino acid derivative, or the protein or the peptide, whose amino group is acylated, α-carboxyl group of the aspartic acid at the C-terminus is converted into oxazolone, and β-carboxyl group is esterified, is allowed to react with an alcohol in the presence of acid. At this time, the protein or the peptide newly generated, whose amino group is modified, C-terminal amino acid is lacking, and carboxyl group of the C-terminal amino acid is esterified, and the amino acid derivative are not affected at all. On the other hand, the protein or the peptide, whose amino group is modified, α-carboxyl group of the aspartic acid at the C-terminus is converted into oxazolone, and β-carboxyl group is esterified, is converted into amixture of the protein or the peptide newly generated, whose amino group is modified, C-terminal amino acid is lacking, and carboxyl group of the C-terminal amino acid is esterified, and an aspartic acid whose β-carboxyl group is esterified.

### Fifth Process

Then, the mixture of the protein or the peptide newly generated, whose amino group is modified, C-terminal amino acid is lacking, and carboxyl group of the C-terminal amino acid is esterified, and the amino acid derivative, or the mixture of the protein or the peptide newly generated, whose amino group is modified, C-terminal amino acid is lacking, and carboxyl group of the C-terminal amino acid is esterified, and the aspartic acid, whose β-carboxyl group is esterified, is allowed to react with an amine, for instance an amine represented by a general formula; NR1R2R3 to give 21 mixture of a protein or a peptide, whose amino acid is modified and C-terminal amino acid is lacking, and the amino acid derivative, or a mixture of a protein or a peptide, whose amino acid is modified and C-terminal amino acid is lacking, and aspartic acid. This aspartic acid is the original C-terminal amino acid.

The amino acid or amino acid derivative obtained in the first to fifth processes described above is isolated and identified to determine the C-terminal amino acid of the protein or the peptide.

Then a series of the operations of the first to fifth processes using the protein or the peptide, whose amino group is modified and C-terminal amino acid is lacking, and the operation of isolating and identifying the amino acid or amino acid derivative obtained is repeatedly carried out to analyse the amino acid sequence of the protein or the peptide from the C-terminus.

### Example 31

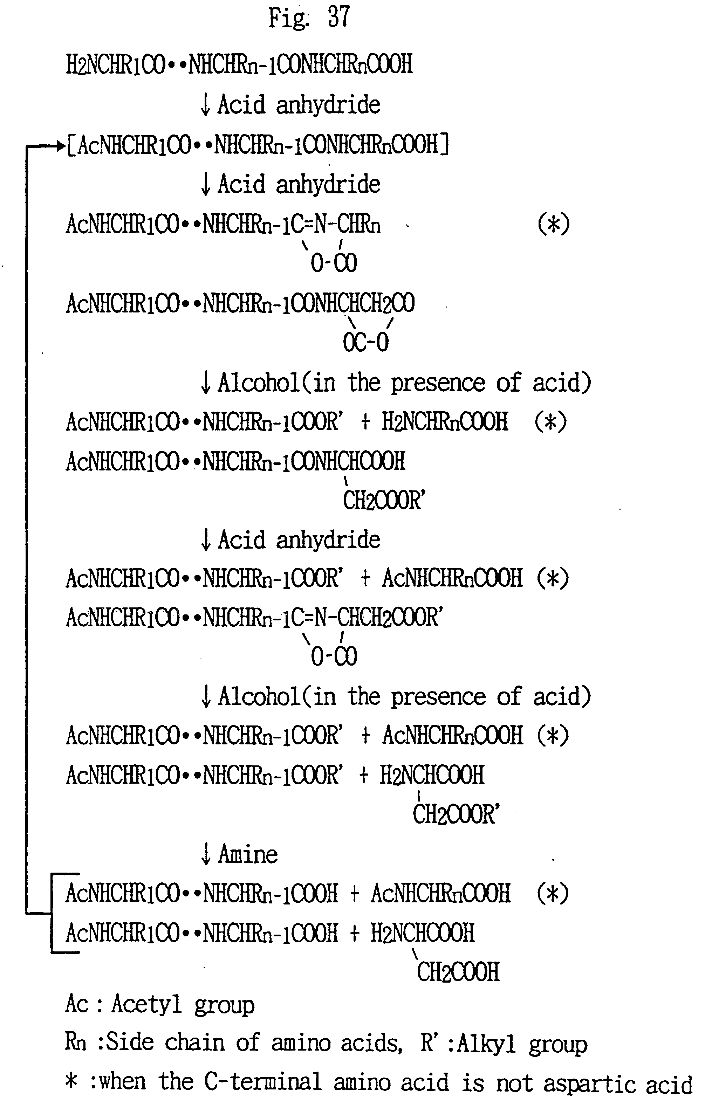

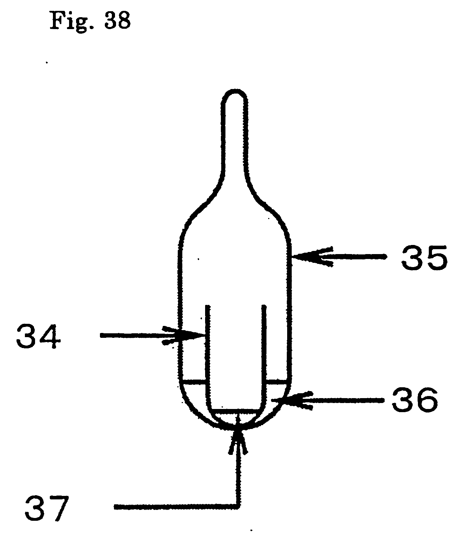

Here, an example of the operation procedure of the present invention is described which analyses an amino acid sequence of a protein or a peptide from a C-terminus thereof by repeating a series of the operations of the first to fifth processes using a protein or a peptide, an operation of isolating and identifying the amino acid from the mixture obtained in the second process, and an operation of isolating and identifying the amino acid from the mixture obtained in the fifth process. Fig. 37 shows a reaction scheme illustrating the experimental procedure of the present invention. Fig. 38 shows an experimental method of the present invention.

### First Process

A sample is allowed to react with acetic anhydride. At first, a sample solution containing a protein or a peptide sample is placed into a small test tube 34 and dried. On the other hand, a solution of a reaction reagent (Here, an acetonitrile solution of acetic anhydride) 36 is placed into a test tube 35. The small test tube 34 having the sample 37 is placed into the test tube 35. This test tube 35 is sealed under reduced pressure with a vacuum pump, and the reaction proceeds.

The reaction conditions are as follows.

| Reaction conditions | |
|---|---|
| Concentration of acetic anhydride | 20% (acetonitrile solution) |
| Reaction temperature | 60°C |
| Reaction period | 10 minutes |

After this reaction, the top of the test tube 35 is opened and the small test tube 34 is taken out. Then the reaction mixture is evaporated under reduced pressure to dryness to remove the reagent and solvent.

### Second Process

The sample obtained thereby is allowed to react with methanol in the presence of HFBA. The experimental procedure and apparatuses used for the reaction are the same as in the first process.

The reaction conditions are as follows.

| Reaction conditions | |
|---|---|
| Concentration of HFBA | 5% (methanol solution) |
| Reaction temperature | 5°C |
| Reaction period | 30 minutes |

After this reaction, the top of the test tube is opened and the small test tube is taken out. Then the reaction mixture is evaporated under reduced pressure to dryness to remove the reagent and solvent.

### Third Process

The sample obtained thereby is allowed to react with acetic anhydride. The experimental procedure and apparatuses used for the reaction are the same as in the first process.

### Fourth Process

The sample obtained thereby is allowed to react with methanol in the presence of HFBA. The experimental procedure and apparatuses used for the reaction are the same as in the second process.

### Fifth Process

The sample obtained thereby is allowed to react with DMAE. The experimental procedure and apparatuses used for the reaction follow in the first process. The solution placed into the test tube is an aqueous solution of DMAE.

The reaction conditions are as follows.

| Reaction conditions | |
|---|---|
| Concentration of DMAE | 50% (aqueous solution) |
| Reaction temperature | 50°C |
| Reaction period | 30 minutes |

In a series of these operations, after the second process, the amino acid is isolated from the sample obtained and identified. Furthermore, after the fifth process, the amino acid is isolated from the sample obtained and identified.

Then, a series of the procedures of the first to fifth processes using the protein or the peptide whose amino group is modified and C-terminal amino acid is lacking, the operation of isolating and identifying the amino acid obtained in the second process, the operation of isolating and identifying the amino acid obtained in the fifth process, and an operation of removing the residual amino acid and amino acid derivative are repeatedly carried out to analyse the amino acid sequence of the protein or the peptide from the C-terminus.

### Example 32

Here, an example of the operation procedure of the present invention is described which analyses an amino acid sequence of a protein or a peptide from a C-terminus thereof by repeating a series of the operations of the first to fifth processes using a protein or a peptide and an operation of isolating and identifying the amino acid from the mixture obtained in the second process.

A series of the operations of the first to fifth processes is the same as in Example 31. In a series of these operations, after the second process, the amino acid is isolated from the sample obtained and identified. Furthermore, after the fifth process, the residual amino acid and amino acid derivative are removed. Then, a series of the procedures of the first to fifth processes using the protein or the peptide whose amino group is modified and C-terminal amino acid is lacking, the operation of isolating and identifying the amino acid obtained in the second process, and the operation of removing the residual amino acid and amino acid derivative are repeatedly carried out to analyse the amino acid sequence of the protein or the peptide from the C-terminus.

### Example 33

Here, an example of the operation procedure of the present invention is described which analyses an amino acid sequence of a protein or a peptide from a C-terminus thereof by repeating a series of the operations of the first to fifth processes using a protein or a peptide and an operation of isolating and identifying the amino acid derivative from the mixture obtained in the third process.

A series of the operations of the first to fifth processes is the same as in Example 31. In a series of these operations, after the third process, the amino acid derivative is isolated from the sample obtained and identified. Furthermore, after the fifth process, the residual amino acid and amino acid derivative are removed. Then, a series of the procedures of the first to fifth processes using the protein or the peptide whose amino group is modified and C-terminal amino acid is lacking, the operation of isolating and identifying the amino acid derivative obtained in the third process, and the operation of removing the residual amino acid and amino acid derivative are repeatedly carried out to analyse the amino acid sequence of the protein or the peptide from the C-terminus.

### Example 34

Here, an example of the operation procedure of the present invention is described which analyses an amino acid sequence of a protein or a peptide from a C-terminus thereof by repeating a series of the operations of the first to fifth processes using a protein or a peptide and an operation of isolating and identifying the amino acid derivative from the mixture obtained in the fourth process.

A series of the operations of the first to fifth processes is the same as in Example 31. In a series of these operations, after the fourth process, the amino acid derivative is isolated from the sample obtained and identified. Furthermore, after the fifth process, the residual amino acid and amino acid derivative are removed. Then, a series of the procedures of the first to fifth processes using the protein or the peptide, whose amino group is modified and C-terminal amino acid is lacking, the operation of isolating and identifying the amino acid derivative obtained in the fourth process, and the operation of removing the residual amino acid and amino acid derivative are repeatedly carried out to analyse the amino acid sequence of the protein or the peptide from the C-terminus.

### Example 35

Here, an example of the operation procedure of the present invention is described which analyses an amino acid sequence of a protein or a peptide from a C-terminus thereof by repeating a series of the operations of the first to fifth processes using a protein or a peptide and an operation of isolating and identifying the amino acid and amino acid derivative from the mixture obtained in the fifth process.

A series of the operations of the first to fifth processes is the same as in Example 31. In a series of these operations, after the fifth process, the amino acid and amino acid derivative are isolated from the sample obtained and identified. Then, a series of the procedures of the first to fifth processes using the protein or the peptide whose amino group is modified and C-terminal amino acid is lacking, and the operation of isolating and identifying the amino acid and amino acid derivative obtained in the fifth process are repeatedly carried out to analyse the amino acid sequence of the protein or the peptide from the C-terminus.

### Example 36

Here, generation of oxazolone in the first process is described.

As the sample, a pentapeptide of Sequence No.1, leucyltryptophanylmethionylarginylphenylalanine (Leu-Trp-MetArg-Phe), was used.

The oxazolone was detected using the cleavage reaction easily caused by alcohol.

The sample obtained in the first process described in Example 31 was allowed to react with methanol.

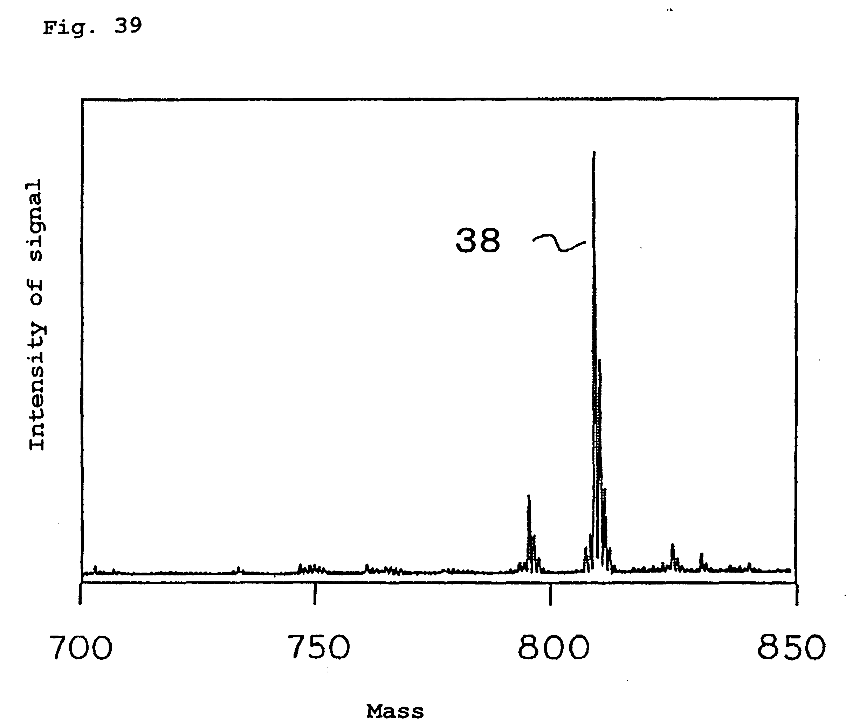

Fig. 39 shows a result of mass spectrometric analysis of the sample. The conditions of mass spectrometric analysis were as follows.

| Conditions of mass spectrometric analysis | |
|---|---|
| Analyzer: | Double focusing mass spectrometer HX-110 (Nihon Denshi) |
| Analysis conditions: | Accelerating potential 10kV |
| Resolution | 1,000 |
| Ion source | FAB (fast-atom bombardment method) |
| Ionization gas | Xe |
| Ion mode | cationic |
| FAB gun accelerating potential | 6kV |
| Detector | MULTIPLIER |
| Load potential | -20kV |
| | |
| Data processing system DA5000 | |
| Matrix | |
| glycerol: thioglycerol :m-nitrobenzyl alcohol=1:1:1 | |

### Procedure for sample preparation

(1) Evaporate the sample under reduced pressure to dryness.
(2) Dissolve the residue in 67% acetic acid (or dimethylformamide) aqueous solution.
(3) Place the matrix of 1µl on the target.
(4) Place the sample solution of 1µl on the target and allow them to mix.
(5) Introduce the mixture into the ion source.

Here, a molecular ion (reference numeral 38) corresponding to the reaction product, whose amino terminus was acetylated and the carboxyl group at the C-terminus was esterified with a methyl group (the methyl ester of acetylleucyltriptophanylmethionylarginylphenylalanine, molecular weight of 808; hereinafter referred to as Ac-LWMRF-OMe) was detected to indicate existence of oxazolone in the sample obtained in the first process of Example 31.

### Example 37

Here, cleavage of the oxazolone ring in the second process is described.

As the sample, a pentapeptide of Sequence No.1, leucyltryptophanylmethionylarginylphenylalanine (Leu-Trp-MetArg-Phe), was used in the same manner as in Example 35.

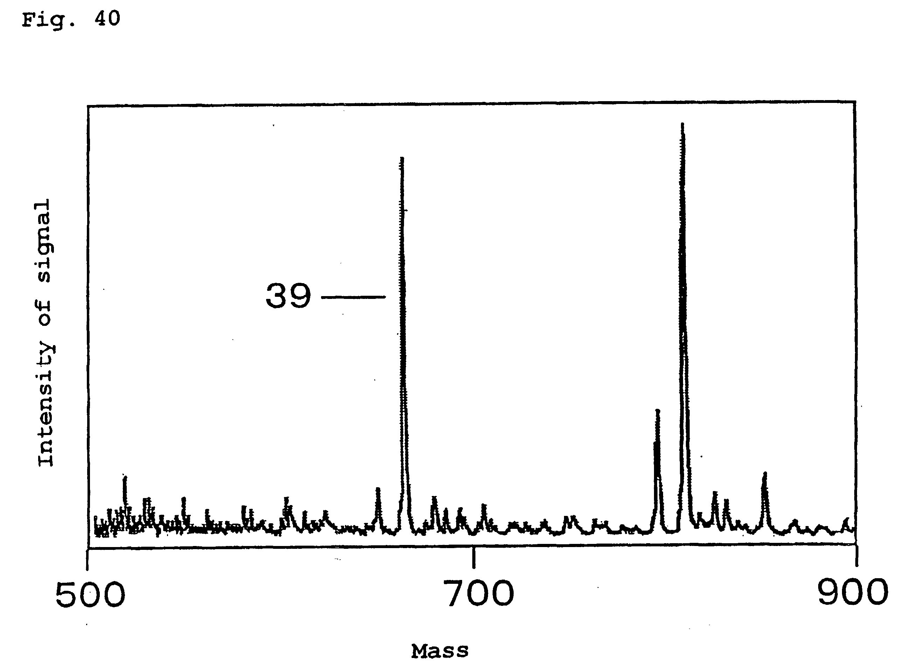

Fig. 40 shows a result of mass spectrometric analysis of the sample obtained in the first and second processes described in Example 31. Here, the sample obtained in the first process was allowed to react with methanol in the presence of HFBA as the second process. The experimental procedure and apparatuses used for the reaction followed in the first process.

The reaction conditions were as follows.

| Reaction conditions | |
|---|---|
| Concentration of HFBA | 5% (methanol solution) |
| Reaction temperature | 25°C |
| Reaction period | 30 minutes |

The conditions for mass spectrometric analysis are the same as in Example 36.

A molecular ion (reference numeral 39) corresponding to the reaction product newly generated, whose amino terminus was acetylated, phenylalanine, C-terminal residue, was eliminated, and the carboxyl group at the C-terminus was esterified with a methyl group (Ac-LWMR-OMe, molecular weight of 661); hereinafter referred to as Ac-LWMRF-OMe) was detected to indicate cleavage of the oxazolone ring in the second process described in Example 2.

### Example 38

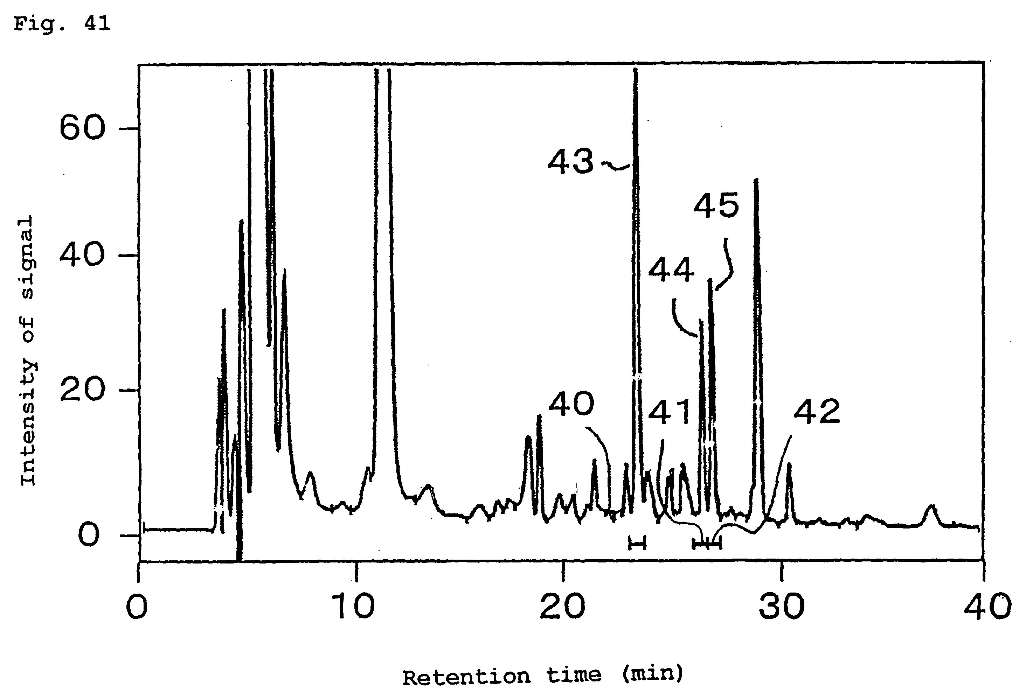

Here, cleavage of the oxazolone ring in the second process is described using a combination of HPLC and mass spectrometry. Fig. 41 shows a result of HPLC analysis of the reaction product obtained in the first and second processes described in Example 31. Here, the sample obtained in the first process was allowed to react with ethanol in the presence of HFBA as the second process. The experimental procedure and apparatuses used for the reaction followed in the first process.

The reaction conditions were as follows.

| Reaction conditions | |
|---|---|
| Concentration of HFBA | 5% (ethanol solution) |
| Reaction temperature | 25°C |
| Reaction period | 30 minutes |

The conditions for HPLC analysis and procedure of preparing the sample were as follows.

### Conditions for HPLC analysis

### Analysis conditions

- Column :: C18 MICROBORE (Shiseido)
- Elution conditions:: Gradient elution with the following solution A and B.
Solution A: 0.1% TEA aqueous
Solution B: 80% acetonitrile aqueous containing
0.1% TEA
- Retention Time: Composition of Eluent
- 0-5 mm: Solution A, 80%
- 5-25 mm: Solution A, 80→40%
(linear gradient)
- 25-30 mm: Solution A, 40%

### Preparation procedure of sample:

(1) Evaporate the sample under reduced pressure to dryness.
(2) Dissolve the residue in 0.1% TEA

Each peak was confirmed to correspond to each product as follows. Fraction 1 to 3 (corresponding to Reference numerals 40 to 42) in Fig. 41 was analysed by mass spectrometry. The conditions for mass spectrometric analysis were as described in Example 36.

In the figure, numeral 40 shows a fraction 1 separated by HPLC from the reaction product obtained in the first and second processes of Example 2. Numeral 41 shows a fraction 2 separated by HPLC from the reaction product obtained in the first and second processes of Example 2. Numeral 42 shows a fraction 3 separated by HPLC from the reaction product obtained in the first and second processes of Example 2. Numeral 43 shows a peak corresponding to the reaction product newly generated of the pentapeptide of Sequence No.1 of which the amino terminal amino acid is acetylated and the phenylalanine, the C-terminal residue, is eliminated, and the carboxyl group at the C-terminus is esterified with an ethyl group (molecular weight of 675). Numeral 44 shows a peak corresponding to the reaction product of the pentapeptide of Sequence No.1 of which the amino terminal amino acid is acetylated (molecular weight of 794). Numeral 45 shows a peak corresponding to the reaction product of the pentapeptide of Sequence No.1 of which the amino terminal amino acid is acetylated (molecular weight of 794).

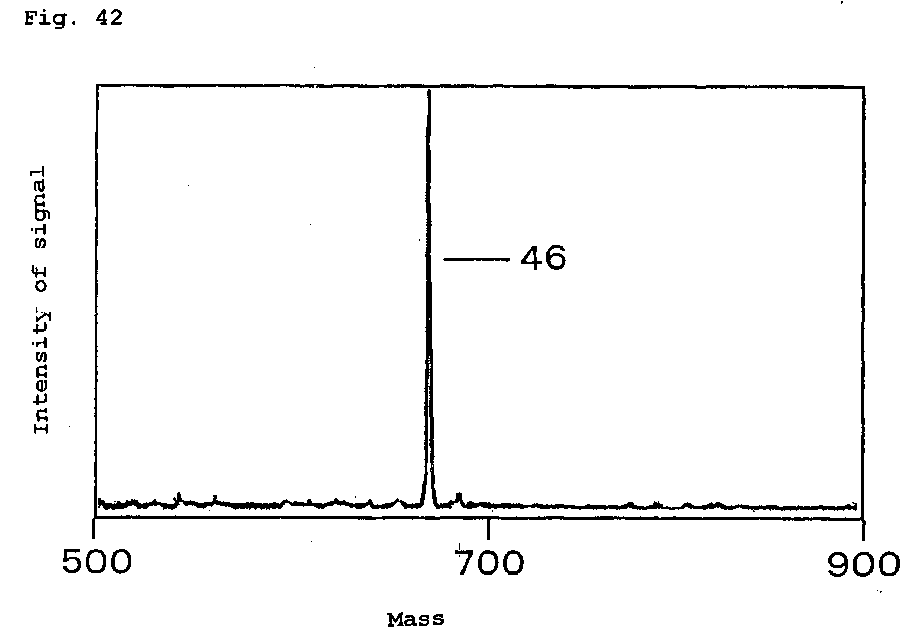

Fig. 42 shows the result of Fraction 1 (Reference numeral 40). Here, Signal 46 from a molecular ion corresponding to the reaction product newly generated, whose amino terminal was acetylated, phenylalanine, C-terminal residue, was eliminated, and the carboxyl group at the C-terminus was esterified with an ethyl group (Ac-LWMR-OEt, molecular weight of 675) was detected. This result also indicates cleavage of the oxazolone ring in the second process of Example 31.

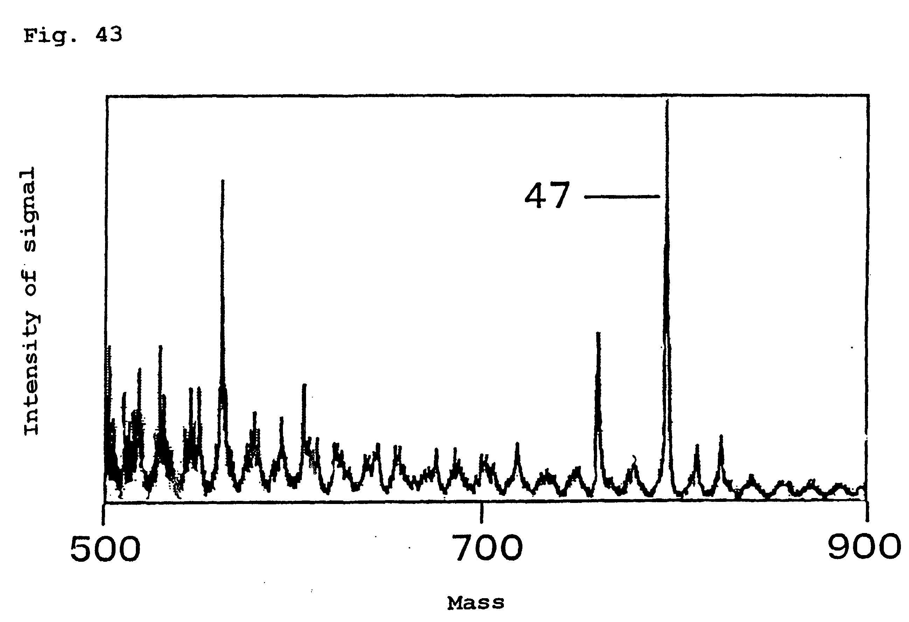

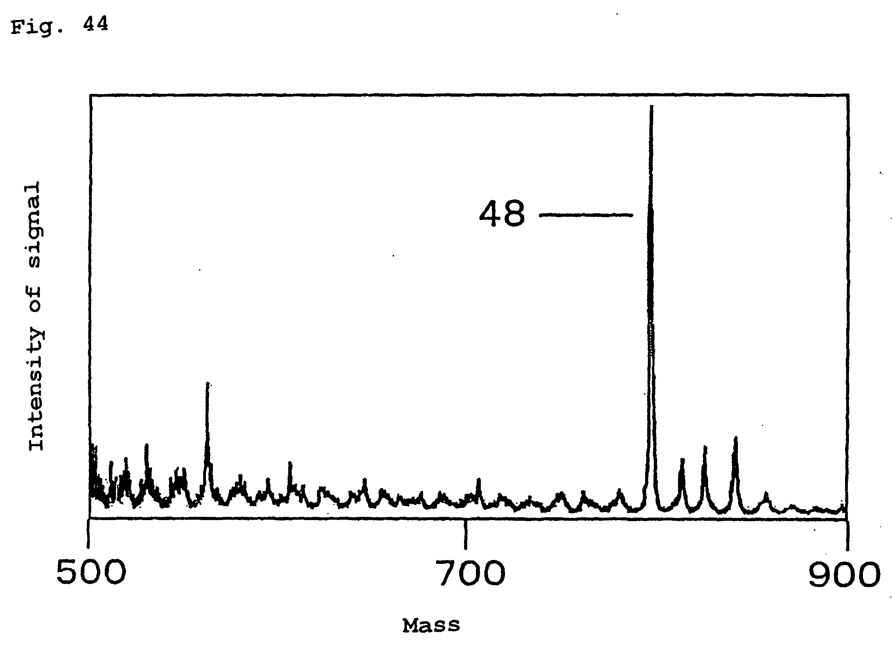

Fig. 43 shows a result of mass spectrometric analysis of Fraction 2 (Reference numeral 41). Fig. 44 shows a result of mass spectrometric analysis of Fraction 3 (Reference numeral 42). determination of these fractions, Signal 47 and 48 from molecular ions of the pentapeptide of Sequence No.1, whose amino terminal was acetylated (acetylleucyltriptophanylmethionylarginylphenylalanine, molecular weight of 794; hereinafter referred to as Ac-LWMRF-OH) was detected in each fraction. This seems to indicate racemization in generation of oxazolone in the first process. These results also indicate generation of an oxazolone ring in the first process and cleavage of the oxazolone ring in the second process.

### Example 39

Here, hydrolysis of the ester in the fifth process is described.

As the sample, a pentapeptide of Sequence No.1, leucyltryptophanylmethionylarginylphenylalanine (Leu-Trp-MetArg-Phe), was used in the same manner as in Example 36.

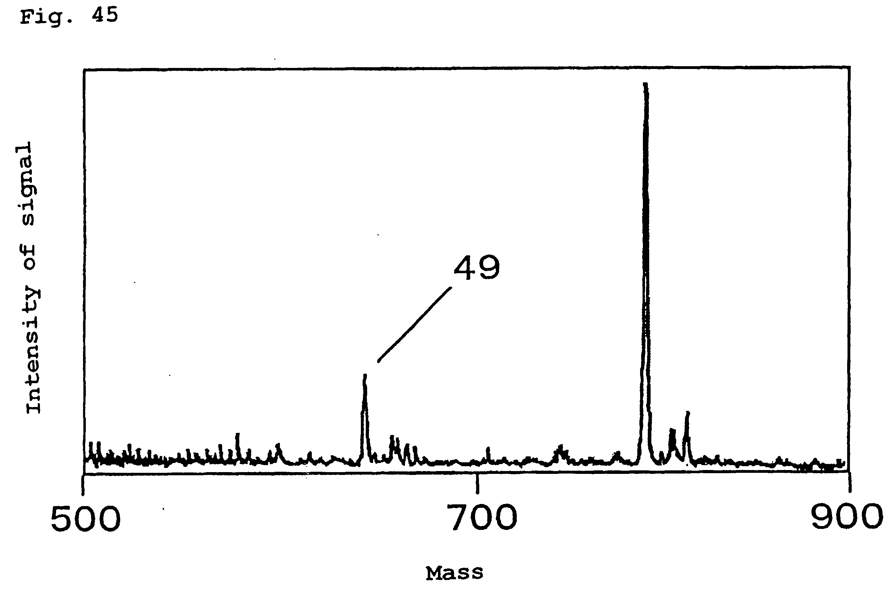

Fig. 45 shows a result of mass spectrometric analysis of the sample obtained in a series of the operations of the first to fifth processes described in Example 31. The conditions for mass spectrometric analysis were the same as in Example 36. Here, a molecular ion (reference numeral 49) corresponding to the reaction product newly generated, whose amino terminus was acetylated, the phenylalanine, the C-terminal residue, was eliminated, and the methyl ester of carboxyl group at the C-terminus was converted into a free carboxyl group (acetylleucyltriptophanylmethionylargininei molecular weight of 647; hereinafter referred to as Ac-LWMR-OH) was detected to indicate hydrolysis of the ester in the fifth process of Example 2.

Accordingly, the results of Example 30 to 39 are summarised as follows.

According to the present invention, in order to analyse the amino acid sequence from the C-terminus, a combination of a series of the operations consisting of the following first to fifth processes and the operation of isolating and identifying the amino acid or amino acid derivative obtained was repeatedly carried out.

### First Process

A protein or a peptide is allowed to react with an acid anhydride, the amino terminal of the protein or the peptide is protected, and the amino acid residue at the C-terminus is modified to generate oxazolone or an intramolecular acid anhydride (when the C-terminal amino acid is aspartic acid).

### Second Process

The reaction product in the first process is allowed to react with an alcohol in the presence of acid, the oxazolone contained therein is subjected to alcoholysis (esterification), and the carboxy-terminal amino acid is liberated or the intramolecular acid anhydride is ring-opened by alcoholysis.

### Third Process

The reaction product in the second process is allowed to react with an acid anhydride, the amino acid residue at the C-terminus of the protein or the peptide, whose C-terminal is not blocked contained therein, is modified to generate oxazolone.

### Fourth Process

The reaction product in the third process is allowed to react with an alcohol in the presence of acid, the oxazolone contained therein is subjected to alcoholysis (esterification), and the carboxy-terminal amino acid is liberated.

### Fifth Process

The reaction product in the fourth process is allowed to react with an amine and the ester contained therein is hydrolyzed.

The present invention enables analysis of an amino acid sequence of a protein or a peptide from a C-terminus thereof without using enzymes by repeating a combination of a series of operations consisting of the processes described above and an operation of isolating and identifying the amino acid obtained, so that it prevents contamination by amino acids and peptides thereof by using enzymes and provides sequential determination which is suitable for microanalyses. Also the present invention provides cleavage of peptide bonds desired and accurate sequencing analysis.

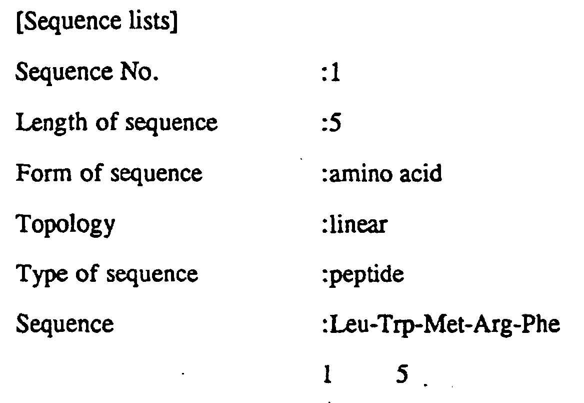

## Claims

1. A method for amino acid sequencing of a protein or a peptide from a carboxy terminus thereof comprising repeating a combined procedure including a process of liberating a carboxy-terminal amino acid of a protein or a peptide and an operation of isolating and identifying the amino acid or amino acid derivative obtained, wherein said combined procedure comprises a first process of allowing the protein or the peptide to react with an acid anhydride and modifying the carboxy terminus to generate oxazolone, including protecting the amino terminal in the operation of said first process; and a second process of allowing the protein or the peptide generated, of which the carboxy terminus is oxazolone, to react with a) acid and alcohol, b) a solution containing acid and alcohol, or c) an ester, to liberate the carboxy-terminal amino acid and an operation of isolating and identifying the amino acid obtained in the second process.

2. A method as claimed in claim 1, further including repeatedly carrying out a combined procedure of a series of operations comprising said first process, a second process of reacting with an alcohol in the presence of acid to liberate the carboxy-terminal amino acid, and a third process of reacting with an amine or an aqueous solution thereof to hydrolyze an ester, and an operation of isolating and identifying the amino acid a) obtained in the second process, or b) after the third process.

3. A method as claimed in claim 1 or 2, in which said first process is carried out in the presence of an acetic acid.

4. A method for amino acid sequencing of a protein or a peptide from a carboxy terminus thereof, **characterised by** repeatedly carrying out a combined procedure of a series of operations comprising a first process of allowing a protein or a peptide to react with an acid anhydride, protecting an amino group thereof, and modifying the carboxy terminus to generate oxazolone when the carboxy-terminal amino acid is not aspartic acid or to give an intramolecular acid anhydride when it is aspartic acid, a second process of reacting with an alcohol in the presence of acid to liberate the carboxy-terminal amino acid when the carboxy terminal is oxazolone or to ring-open the intramolecular acid anhydride by alcoholysis when it is an intramolecular acid anhydride, a third process of reacting with an acid anhydride to form oxazolone at the carboxy terminal, a fourth process of reacting with an alcohol to liberate the carboxy-terminal amino acid as an ester of the amino acid, and a fifth process of reacting with an amine to hydrolyze the ester and an operation of isolating and identifying a) the amino acid obtained in the second process, and/or the fifth process, or b) the amino acid derivative obtained in the third, fourth or fifth process.

5. A method as claimed in claim 4, in which the fourth process of reacting with an alcohol to liberate the carboxy-terminal amino acid as an ester of the amino acid is carried out in the presence of acid.

## Patentansprüche

1. Verfahren zur Bestimmung der Aminosäuresequenz eines Proteins oder eines Peptids, beginnend mit dessen Carboxylende, umfassend:
Wiederholen einer kombinierten Vorgehensweise, die ein Verfahren, in dem eine carboxyterminale Aminosäure eines Proteins oder eines Peptids freigesetzt wird und einen Vorgang, in dem die erhaltene Aminosäure oder das erhaltene Aminosäurederivat isoliert und identifiziert wird beinhaltet,
wobei die kombinierte Vorgehensweise umfasst:
ein erstes Verfahren, in welchem ermöglicht wird, dass das Protein oder das Peptid mit einem Säureanhydrid reagiert und in welchem das Carboxylende modifiziert wird um Oxazolon zu bilden, einschließlich Schützen des Aminoendes während des ersten Verfahrens; und
ein zweites Verfahren, in welchem ermöglicht wird, dass das gebildete Protein oder Peptid dessen Carboxylende Oxazolon ist, mit a) Säure und Alkohol, b) einer Lösung die Säure und Alkohol enthält oder c) einem Ester reagiert, um die carboxyterminale Aminosäure freizusetzen; und
einen Vorgang, in dem die in dem zweiten Verfahren erhaltene Aminosäure isoliert und identifiziert wird.

2. Verfahren nach Anspruch 1, welches weiterhin das wiederholte Ausführen einer kombinierten Vorgehensweise aus einer Reihe von Vorgängen umfasst, umfassend: das erste Verfahren, ein zweites Verfahren, in dem eine Umsetzung mit einem Alkohol in Gegenwart von Säure erfolgt, um die carboxyterminale Aminosäure freizusetzen und ein drittes Verfahren, in dem eine Umsetzung mit einem Amin oder einer wässrigen Lösung davon erfolgt, um einen Ester zu hydrolysieren und einen Vorgang, in dem die a) in dem zweiten Verfahren oder b) nach dem dritten Verfahren erhaltene Aminosäure identifiziert und isoliert wird.

3. Verfahren nach Anspruch 1 oder 2, worin das erste Verfahren in Gegenwart einer Essigsäure durchgeführt wird.

4. Verfahren zur Bestimmung der Aminosäuresequenz eines Proteins oder eines Peptids, beginnend mit dessen Carboxylende, **gekennzeichnet durch** das wiederholte Durchführen einer kombinierten Vorgehensweise aus einer Reihe von Vorgängen, umfassend:
ein erstes Verfahren, in dem es einem Protein oder einem Peptid ermöglicht wird, mit einem Säureanhydrid zu reagieren, Schützen einer Aminogruppe davon und Modifizieren des Carboxylendes um Oxazolon zu bilden, wenn die carboxyterminale Aminosäure nicht Aspartamsäure ist oder um ein intramolekulares Säureanhydrid zu bilden, wenn sie Aspartamsäure ist,
ein zweites Verfahren in dem eine Umsetzung mit einem Alkohol in Gegenwart von Säure erfolgt, um die carboxyterminale Aminosäure freizusetzen, wenn das Carboxylende Oxazolon ist oder um eine Ringöffnung des intramolekularen Säureanhydrids **durch** Alkoholyse zu bewirken, wenn es ein intramolekulares Säureanhydrid ist,
ein drittes Verfahren, in dem eine Umsetzung mit einem Säureanhydrid erfolgt, um Oxazolon an dem Carboxylende zu bilden,
ein viertes Verfahren in dem eine Umsetzung mit einem Alkohol erfolgt, um die carboxyterminale Aminosäure als einen Ester der Aminosäure freizusetzen und
ein fünftes Verfahren in dem eine Umsetzung mit einem Amin erfolgt, um den Ester zu hydrolysieren, und
einen Vorgang, in dem a) die in dem zweiten und/oder dem fünften Verfahren erhaltene Aminosäure oder b) das in dem in dem dritten, vierten oder fünften Verfahren erhaltene Aminosäurederivat identifiziert und isoliert wird.

5. Verfahren nach Anspruch 4, worin das vierte Verfahren, in dem eine Umsetzung mit einem Alkohol erfolgt, um die carboxyterminale Aminosäure als einen Ester der Aminosäure freizusetzen, in Gegenwart einer Säure durchgeführt wird.

## Revendications

1. Procédé pour le séquençage d'acides aminés d'une protéine ou d'un peptide à partir d'une extrémité carboxy de celui-ci, comprenant la répétition d'une procédure combinée englobant un procédé de libération d'un acide aminé carboxy-terminal d'une protéine ou d'un peptide, et une opération d'isolation et d'identification de l'acide aminé ou du dérivé d'acide aminé obtenu, dans lequel ladite procédure combinée comprend un premier processus consistant à permettre à la protéine ou au peptide de réagir avec un anhydride d'acide et à modifier l'extrémité carboxy pour générer une oxazolone, comprenant la protection de l'extrémité amino dans l'opération dudit premier processus ; et un deuxième processus consistant à permettre à la protéine ou au peptide généré, dont l'extrémité carboxy est une oxazolone, de réagir avec a) un acide et un alcool, b) une solution contenant un acide et un alcool, ou c) un ester, pour libérer l'acide aminé carboxy-terminal, et une opération d'isolation et d'identification de l'acide aminé obtenu dans le deuxième processus.

2. Procédé selon la revendication 1, comprenant en outre la mise en oeuvre répétée d'une procédure combinée d'une série d'opérations comprenant ledit premier processus, un deuxième processus de réaction avec un alcool en présence d'acide pour libérer l'acide aminé carboxy-terminal, et un troisième processus de réaction avec une amine ou une solution aqueuse de celle-ci pour hydrolyser un ester, et une opération d'isolation et d'identification de l'acide aminé a) obtenu dans le deuxième processus, ou b) après le troisième processus.

3. Procédé selon la revendication 1 ou 2, dans lequel ledit premier processus est mis en oeuvre en présence d'un acide acétique.

4. Procédé pour le séquençage d'acides aminés d'une protéine ou d'un peptide à partir d'une extrémité carboxy de celui-ci, **caractérisé par** la mise en oeuvre répétée d'une procédure combinée d'une série d'opérations comprenant un premier processus consistant à permettre à une protéine ou un peptide de réagir avec un anhydride d'acide, à protéger un groupe amino de celui-ci, et à modifier l'extrémité carboxy pour générer une oxazolone quand l'acide aminé carboxy-terminal n'est pas l'acide aspartique, ou pour donner un anhydride d'acide intramoléculaire quand il s'agit de l'acide aspartique, un deuxième processus de réaction avec un alcool en présence d'acide pour libérer l'acide aminé carboxy-terminal quand l'extrémité carboxy est une oxazolone ou d'ouvrir le cycle de l'anhydride d'acide intramoléculaire par alcoolyse quand il s'agit d'un anhydride d'acide intramoléculaire, un troisième processus de réaction avec un anhydride d'acide pour former une oxazolone à l'extrémité carboxy, un quatrième processus de réaction avec un alcool pour libérer l'acide aminé carboxy-terminal sous la forme d'un ester de l'acide aminé, et un cinquième processus de réaction avec une amine pour hydrolyser l'ester, et une opération d'isolation et d'identification a) de l'acide aminé obtenu dans le deuxième processus et/ou le cinquième processus, ou b) du dérivé d'acide aminé obtenu dans le troisième, le quatrième ou le cinquième processus.

5. Procédé selon la revendication 4, dans lequel le quatrième processus de réaction avec un alcool pour libérer l'acide aminé carboxy-terminal sous la forme d'un ester de l'acide aminé est mis en oeuvre en présence d'acide.
